# EUROPEAN PATENT APPLICATION

(11) **EP 3 895 682 A1**
(43) Date of publication of application: **20.10.2021**
(21) Application number: 19895945.4
(22) Date of filing: 12.12.2019
(51) Int. Cl.: A61K 6/831, A61K 6/84, A61K 6/887

(54) **DENTAL COMPOSITION**

(30) Priority: 13.12.2018 JP 2018233875
(71) Applicant: Kuraray Noritake Dental Inc., Kurashiki-shi, Okayama 710-0801 (JP)
(72) Inventor: KAJIKAWA Tatsuya, Tainai-shi, Niigata 959-2653 (JP); HORIGUCHI Hirotaka, Tainai-shi, Niigata 959-2653 (JP)
(74) Representative: D Young & Co LLP
(86) International application number: PCT/JP2019/048764
(87) International publication number: WO 2020/122192

(57) **Abstract**

The present invention provides a dental composition, suitable for use as a dental composite resin or other dental material, having good ease of handling, and that is highly stable in consistency and low in polymerization shrinkage stress while providing desirable mechanical strength and polishability for the cured product. The present invention relates to a dental composition comprising a polymerizable monomer (A), an inorganic filler (B), a prepolymer (C), and a polymerization initiator (D), the inorganic filler (B) having an average particle diameter of 1 to 20 µm, and a specific surface area of 10 to 300 m²/g. The prepolymer (C) preferably has a structure derived from the polyfunctional monomer (a). The polyfunctional monomer (a) has a molecular weight of preferably 250 to 1,000.

## Description

### TECHNICAL FIELD

The present invention relates to a dental composition that can be suitably used in dental applications such as dental materials, particularly dental composite resins, for partial or whole replacement of natural tooth.

### BACKGROUND ART

A dental composite resin, a mixture of primarily polymerizable monomer, filler, and polymerization initiator, is a dental material that has become the material of choice for restoration of missing tooth parts and cavities. Dental composite resins are required to satisfy the following properties. Specifically, a dental composite resin, in the form of a cured article after polymerization and cure, must satisfy requirements such as a mechanical strength high enough to replace natural teeth, and polishability (gloss polishability) that enables the surface to be readily finished to high gloss after a short polishing time. Before polymerization and cure, a dental composite resin itself (usually in paste form) must satisfy a number of requirements, including good ease of handling (for example, the properties allowing clinicians and denturists to easily handle the resin without the paste adhering to dental instruments or being too sticky).

These properties are greatly influenced by the filler used. For example, when an inorganic filler with a relatively large particle diameter is used, the mechanical strength of cured product and the ease of handling of dental composite resin can improve with relative ease because larger fillers enable a dental composite resin to have a higher filler content. The downside, however, is that the surface cannot be polished to a sufficient gloss even after finishing. A cured article can exhibit improved gloss polishability by using an inorganic filler having a relatively small particle diameter. However, in this case, the dental composite resin tends to have increased viscosity, and cannot easily increase its filler content. This often leads to a cured product having low mechanical strength, or a dental composite resin with poor ease of handling.

Various methods have been studied to provide a solution to these problems. For example, Patent Literature 1 describes a dental composition comprising silica fine particles, and a specific oxide covering the surface of the silica fine particles. It is stated in this related art document that the dental composition, with the use of a specific filler having an average particle diameter of 1 to 20 µm and a specific surface area of 50 to 300 m²/g, can provide a dental composite resin having desirable properties, including improved mechanical strength and polishability for the cured product, and improved ease of handling for the paste.

### CITATION LIST

### Patent Literature

Patent Literature 1: JP 2009-286783 A

### SUMMARY OF INVENTION

### Technical Problem

However, studies found that a dental composition using an inorganic filler having the average particle diameter and specific surface area specified in Patent Literature 1 tends to undergo changes in consistency over time. A shrinkage that occurs during polymerization and curing of a dental composition often causes the cured product to pull away, and creates a contraction gap, which often leads to problems such as secondary caries, a stimulation of the pulp, staining, and detachment of the cured product. The dental composition described in Patent Literature 1 experiences a relatively large stress during polymerization and contraction (hereinafter, referred to as "polymerization shrinkage stress"), and has been found to easily contract during polymerization and cure. Further improvements are needed in this regard.

It is accordingly an object of the present invention to provide a dental composition, suitable for use as a dental composite resin or other dental material, having good ease of handling, and that is highly stable in consistency and low in polymerization shrinkage stress while providing desirable mechanical strength and polishability for the cured product.

### Solution to Problem

The present inventors conducted intensive studies to achieve the foregoing object, and found that a dental composite resin using an inorganic filler having a specified average particle diameter and a specified specific surface area, when additionally containing a prepolymer, can have good ease of handling, and becomes highly stable in consistency and low in polymerization shrinkage stress while providing desirable mechanical strength and polishability for the cured product. The present invention was completed after further studies based on this finding.

Specifically, the present invention relates to the following.
[1] A dental composition comprising a polymerizable monomer (A), an inorganic filler (B), a prepolymer (C), and a polymerization initiator (D), the inorganic filler (B) having an average particle diameter of 1 to 20 µm, and a specific surface area of 10 to 300 m²/g.
[2] The dental composition according to [1], wherein the prepolymer (C) comprises a structure derived from a polyfunctional monomer (a).
[3] The dental composition according to [2], wherein the polyfunctional monomer (a) has a molecular weight of 250 to 1,000.
[4] The dental composition according to [2] or [3], wherein the polyfunctional monomer (a) comprises at least one selected from the group consisting of a polyfunctional (meth)acrylic acid ester and a polyfunctional (meth)acrylamide.
[5] The dental composition according to any one of [2] to [4], wherein the polyfunctional monomer (a) comprises a compound represented by the following general formula (1):

   X-A-X (1),

   where X is a (meth)acryloyloxy group or a (meth)acrylamide group, and A is an optionally substituted divalent aliphatic hydrocarbon group having 5 or more carbon atoms, or an optionally substituted divalent aromatic hydrocarbon group having 6 or more carbon atoms, wherein some of the constituent carbon atoms in A may be substituted with oxygen atoms and/or nitrogen atoms, and a plurality of X may be the same or different from each other.
[6] The dental composition according to any one of [2] to [5], wherein the polyfunctional monomer (a) comprises a compound represented by the following general formula (2):

   X-(R¹)ₘ-A'-(R²)ₙ-X (2),

   where X is a (meth)acryloyloxy group or a (meth)acrylamide group, R¹ and R² are each independently an optionally substituted alkyleneoxy group having 1 to 6 carbon atoms, and A' is an optionally substituted divalent aliphatic hydrocarbon group having 3 or more carbon atoms, or an optionally substituted divalent aromatic hydrocarbon group having 6 or more carbon atoms, wherein some of the constituent carbon atoms in A' may be substituted with oxygen atoms and/or nitrogen atoms, m and n are each independently an integer of 1 or more, the average number of moles of alkyleneoxy groups added as represented by an average of the sum of m and n per molecule is 2 to 35, and a plurality of X, R¹, and R² each may be the same or different from each other.
[7] The dental composition according to any one of [2] to [6], wherein the prepolymer (C) additionally comprises a structure derived from a monofunctional monomer (b).
[8] The dental composition according to [7], wherein the monofunctional monomer (b) comprises at least one selected from the group consisting of a monofunctional (meth)acrylic acid ester and a monofunctional (meth)acrylamide.
[9] The dental composition according to [7] or [8], wherein at least one of the polyfunctional monomer (a) and the monofunctional monomer (b) comprises an aromatic ring.
[10] The dental composition according to any one of [7] to [9], wherein the structure derived from the polyfunctional monomer (a) and the structure derived from the monofunctional monomer (b) have a mole ratio of 10/90 to 90/10 in terms of a mole ratio of the structure derived from the polyfunctional monomer (a) to the structure derived from the monofunctional monomer (b).
[11] The dental composition according to any one of [1] to [10], wherein the prepolymer (C) has a hydroxyl number of 250 mgKOH/g or less.
[12] The dental composition according to any one of [1] to [11], wherein the prepolymer (C) comprises a structure derived from a chain transfer agent (c).
[13] The dental composition according to any one of [1] to [12], wherein the inorganic filler (B) comprises a secondary particle formed by binding of inorganic primary fine particles.
[14] The dental composition according to any one of [1] to [13], wherein the inorganic filler (B) comprises silicon dioxide and at least one metal oxide other than silicon dioxide.
[15] The dental composition according to [14], wherein the metal oxide other than silicon dioxide is at least one selected from the group consisting of zirconium oxide, barium oxide, lanthanum oxide, and ytterbium oxide.
[16] The dental composition according to any one of [1] to [15], wherein the inorganic filler (B) is surface treated with a silane coupling agent.
[17] The dental composition according to any one of [1] to [16], wherein the dental composition comprises 10 to 97 mass% of the inorganic filler (B).
[18] The dental composition according to any one of [1] to [17], wherein the dental composition comprises 0.5 to 20 mass% of the prepolymer (C).
[19] The dental composition according to any one of [1] to [18], wherein the dental composition is a dental composite resin.

### Advantageous Effects of Invention

According to the present invention, a dental composition, suitable for use as a dental composite resin or other dental material, can be provided that has good ease of handling, and that is highly stable in consistency and low in polymerization shrinkage stress while providing desirable mechanical strength and polishability for the cured product.

### DESCRIPTION OF EMBODIMENTS

The present invention is described below in detail.

### Dental Composition

A dental composition of the present invention comprises a polymerizable monomer (A), an inorganic filler (B), a prepolymer (C), and a polymerization initiator (D). The inorganic filler (B) has an average particle diameter of 1 to 20 µm, and a specific surface area of 10 to 300 m²/g.

A dental composition having such a configuration is suitable for use as a dental composite resin or other dental material, and can have good ease of handling, in addition to being highly stable in consistency and low in polymerization shrinkage stress, and providing desirable mechanical strength and polishability for the cured product.

A possible explanation for these desirable effects is as follows, though this is not to be construed as limiting the present invention in any ways. Specifically, a dental composition using an inorganic filler having an average particle diameter of about 1 to 20 µm and a specific surface area of about 10 to 300 m²/g tends to undergo changes in consistency over time as a result of a polymerizable monomer readily infiltrating the inorganic filler during storage of the dental composition. However, the use of a prepolymer appears to inhibit such infiltration of polymerizable monomer. A prepolymer can prevent a large increase in the elastic modulus of cured product while decreasing the density of functional groups in the dental composition. These are probably responsible for the ability of prepolymer to effectively reduce polymerization shrinkage stress. During polymerization and cure, unreacted polymerizable functional groups in prepolymer (C) react with components of dental composition other than prepolymer (C), and form strong bonds with these components. Presumably, this enables the dental composition to form a cured article having high mechanical strength with no weak portions.

### Polymerizable Monomer (A)

The polymerizable monomer (A) contained in a dental composition of the present invention may be a known polymerizable monomer used for dental compositions. Particularly preferred for use are radical polymerizable monomers. Examples of the radical polymerizable monomers include esters of unsaturated carboxylic acids such as α-cyanoacrylic acid, (meth)acrylic acid, α-halogenated acrylic acid, crotonic acid, cinnamic acid, sorbic acid, maleic acid, and itaconic acid; (meth)acrylamide; derivatives of (meth)acrylamide; vinyl esters; vinyl ethers; mono-N-vinyl derivatives; and derivatives of styrene. The polymerizable monomer (A) may be used alone, or two or more thereof may be used in combination. Preferred are esters of unsaturated carboxylic acids, and derivatives of (meth)acrylamide. More preferred are esters of (meth)acrylic acid, and derivatives of (meth)acrylamide. Even more preferred are esters of (meth)acrylic acid. As used herein, "(meth)acryl" is intended to be inclusive of both "methacryl" and "acryl". Examples of esters of (meth)acrylic acid and derivatives of (meth)acrylamide are as follows.
(i) Monofunctional (Meth)Acrylic Acid Esters and Derivatives of (Meth)Acrylamide
   Examples include methyl (meth)acrylate, isobutyl (meth)acrylate, benzyl (meth)acrylate, dodecyl (meth)acrylate, 2-(N,N-dimethylamino)ethyl (meth)acrylate, 2,3-dibromopropyl (meth)acrylate, 3-(meth)acryloyloxypropyltrimethoxysilane, 11-(meth)acryloyloxyundecyltrimethoxysilane, 2-hydroxyethyl (meth)acrylate, 3-hydroxypropyl (meth)acrylate, 4-hydroxybutyl (meth)acrylate, 6-hydroxyhexyl (meth)acrylate, 10-hydroxydecyl (meth)acrylate, propylene glycol mono(meth)acrylate, glycerin mono(meth)acrylate, erythritol mono(meth)acrylate, phenoxyethylene glycol (meth)acrylate, isobornyl (meth)acrylate, 3-phenoxybenzyl (meth)acrylate, N-methylol (meth)acrylamide, N-hydroxyethyl (meth)acrylamide, N, N-bis(hydroxyethyl)(meth)acrylamide, N,N-dimethyl (meth)acrylamide, N,N-diethyl (meth)acrylamide, N,N-di-n-propyl (meth)acrylamide, N-ethyl-N-methyl (meth)acrylamide, (meth)acryloylmorpholine, (meth)acryloyloxydodecylpyridinium bromide, (meth)acryloyloxydodecylpyridinium chloride, (meth)acryloyloxyhexadecylpyridinium bromide, and (meth)acryloyloxyhexadecylpyridinium chloride.
(ii) Bifunctional (Meth)Acrylic Acid Esters
   Examples include aromatic bifunctional (meth)acrylic acid esters, and aliphatic bifunctional (meth)acrylic acid esters.
   Examples of aromatic bifunctional (meth)acrylic acid esters include 2,2-bis((meth)acryloyloxyphenyl)propane, 2,2-bis[4-(3-acryloyloxy-2-hydroxypropoxy)phenyl]propane, 2,2-bis[4-(3-methacryloyloxy-2-hydroxypropoxy)phenyl]propane (commonly known as Bis-GMA), 2,2-bis(4-(meth)acryloyloxypolyethoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propa ne, 2,2-bis(4-(meth)acryloyloxypropoxyphenyl)propane, 2,2 -bis(4- (meth) acryloyloxyisopropoxyphenyl)prop ane, 2,2-bis(4-(meth)acryloyloxydipropoxyphenyl)propane, 2-(4-(meth)acryloyloxydipropoxyphenyl)-2-(4-(meth)acryloyloxytriethoxyphenyl)prop ane, 9,9-bis[4-(2-(meth)acryloyloxyethoxy)phenyl]fluorene, 9,9-bis[4-(2-(meth)acryloyloxypolyethoxy)phenyl]fluorene, diphenylbis[3-(meth)acryloyloxypropyl]silane, and methylphenylbis[3-(meth)acryloyloxypropyl]silane.
   Examples of aliphatic bifunctional (meth)acrylic acid esters include glycerol di(meth)acrylate, ethylene glycol di(meth)acrylate, diethylene glycol di(meth)acrylate, triethylene glycol diacrylate, triethylene glycol dimethacrylate (commonly known as 3G), propylene glycol di(meth)acrylate, butylene glycol di(meth)acrylate, neopentyl glycol di(meth)acrylate, 1,3-butanediol di(meth)acrylate, 1,5-pentanediol di(meth)acrylate, 1,6-hexanediol di(meth)acrylate, 1,10-decanediol di(meth)acrylate, 1,12-dodecanediol di(meth)acrylate, 1,2-bis(3-(meth)acryloyloxy-2-hydroxypropyloxy)ethane, tricyclodecanedimethanol di(meth)acrylate, 2,2,4-trimethylhexamethylenebis(2-carbamoyloxyethyl)diacrylate, 2,2,4-trimethylhexamethylenebis(2-carbamoyloxyethyl)dimethacrylate (commonly known as UDMA), and dicyclohexylbis[3-(meth)acryloyloxypropyl]silane.
(iii) Tri- and Higher-Functional (Meth)Acrylic Acid Esters
   Examples include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol tri(meth)acrylate, dipentaerythritol tetra(meth)acrylate, dipentaerythritol penta(meth)acrylate, N,N'-(2,2,4-trimethylhexamethylene)bis[2-(aminocarboxy)propane-1,3-diol]tetra(met h)acrylate, and 1,7-di(meth)acryloyloxy-2,2,6,6-tetra(meth)acryloyloxymethyl-4-oxaheptane.

For properties such as improved ease of handling of the dental composition and improved mechanical strength of the cured product, preferred as the (meth)acrylic acid esters and derivatives of (meth)acrylamide are aromatic bifunctional (meth)acrylic acid esters and aliphatic bifunctional (meth)acrylic acid esters, more preferably 2,2-bis[4-(3-acryloyloxy-2-hydroxypropoxy)phenyl]propane, 2,2-bis[4-(3-methacryloyloxy-2-hydroxypropoxy)phenyl]propane (Bis-GMA), 2,2-bis(4-(meth)acryloyloxypolyethoxyphenyl)propane (average number of moles of ethyleneoxy groups added:1 to 30), triethylene glycol diacrylate, triethylene glycol dimethacrylate (3G), 1,10-decanediol di(meth)acrylate, 1,12-dodecanediol di(meth)acrylate, 1,2-bis(3-(meth)acryloyloxy-2-hydroxypropyloxy)ethane, tricyclodecanedimethanol di(meth)acrylate, 2,2,4-trimethylhexamethylenebis(2-carbamoyloxyethyl)diacrylate, and 2,2,4-trimethylhexamethylenebis(2-carbamoyloxyethyl)dimethacrylate (UDMA), even more preferably 2,2-bis[4-(3-methacryloyloxy-2-hydroxypropoxy)phenyl]propane (Bis-GMA), 2,2-bis(4-methacryloyloxypolyethoxyphenyl)propane (average number of moles of ethyleneoxy groups added: 1 to 30), triethylene glycol dimethacrylate (3G), 1,10-decanediol dimethacrylate, 1,12-dodecanediol dimethacrylate, 1,2-bis(3-methacryloyloxy-2-hydroxypropyloxy)ethane, tricyclodecane dimethanol dimethacrylate, and 2,2,4-trimethylhexamethylenebis(2-carbamoyloxyethyl)dimethacrylate (UDMA), particularly preferably 2,2-bis[4-(3-methacryloyloxy-2-hydroxypropoxy)phenyl]propane (Bis-GMA), 2,2-bis(4-methacryloyloxypolyethoxyphenyl)propane (average number of moles of ethyleneoxy groups added of about 2.6), and triethylene glycol dimethacrylate (3G).

In order for a dental composition to have desirable properties such as desirable adhesive properties for adherends such as tooth structure, metal, and ceramic, it may be preferable that the polymerizable monomer (A) contain functional monomers capable of imparting adhesive properties to such adherends.

In view of providing desirable adhesive properties for tooth structure and base metal, the functional monomers are, for example, polymerizable monomers having a phosphoric acid group, such as 2-(meth)acryloyloxyethyl dihydrogen phosphate, 10-(meth)acryloyloxydecyl dihydrogen phosphate, and 2-(meth)acryloyloxyethylphenyl hydrogen phosphate; or polymerizable monomers having a carboxylic acid group, such as 11-(meth)acryloyloxyundecane-1,1-dicarboxylic acid, and 4-(meth)acryloyloxyethoxycarbonylphthalic acid. In view of providing desirable adhesive properties for noble metals, the functional monomers are, for example, 10-mercaptodecyl (meth)acrylate, 6-(4-vinylbenzyl-n-propyl)amino-1,3,5-triazine-2,4-dithione, the derivatives of thiouracil mentioned in JP 10(1998)-1473 A, or the compounds having a sulfur element mentioned in JP 11(1999)-92461 A. In view of effective adhesion for ceramic, porcelain, and other dental compositions, the functional monomers are, for example, silane coupling agents such as γ-(meth)acryloyloxypropyltrimethoxysilane.

The content of polymerizable monomer (A) in a dental composition of the present invention is not particularly limited. However, in view of properties such as ease of handling of the dental composition obtained or the mechanical strength of the cured product, the content of polymerizable monomer (A) is preferably 1 mass% or more, more preferably 2 mass% or more, even more preferably 5 mass% or more, and may be 8 mass% or more, or 15 mass% or more, relative to the mass of the dental composition. The content of polymerizable monomer (A) is preferably 70 mass% or less, more preferably 50 mass% or less, even more preferably 40 mass% or less, particularly preferably 30 mass% or less.

### Inorganic Filler (B)

A dental composition of the present invention comprises an inorganic filler (B) having an average particle diameter of 1 to 20 µm, and a specific surface area of 10 to 300 m²/g.

The average particle diameter of inorganic filler (B) is 1 µm or more, preferably 1.5 µm or more, more preferably 2 µm or more, even more preferably 4 µm or more, and is 20 µm or less, preferably 15 µm or less, more preferably 10 µm or less, even more preferably 7 µm or less. By setting these lower limits for the average particle diameter of inorganic filler (B), the dental composition can effectively have reduced stickiness, and ease of handling improves. By setting the foregoing upper limits for the average particle diameter of inorganic filler (B), the dental composition can effectively have reduced runniness or roughness, and ease of handling improves. When the inorganic filler (B) is an agglomerated particle (secondary particle), the average particle diameter of inorganic filler (B) means an average particle diameter of the agglomerated particle (secondary particle).

A laser diffraction scattering method can be used for the average particle diameter measurement of inorganic filler (B). Specifically, for example, a laser diffraction particle size distribution analyzer (for example, SALD-2100 manufactured by Shimadzu Corporation) may be used with ethanol or a 0.2% sodium hexametaphosphate aqueous solution used as dispersion medium. For the average particle diameter measurement of inorganic filler (B) using such a method, it is preferable that the average particle diameter be measured after ashing the inorganic filler (B), for example, by treating the inorganic filler (B) at 450°C for 4 hours, in order to eliminate the influence of a surface treatment when the inorganic filler (B) is subjected to a surface treatment, as will be described later. Preferably, ashing is performed for the average particle diameter measurement of the inorganic filler (B) contained in the dental composition. The average particle diameter of inorganic filler (B) can be measured using the method specifically described in the EXAMPLES section below.

The specific surface area of inorganic filler (B) is 10 m²/g or more, preferably 20 m²/g or more, more preferably 50 m²/g or more, even more preferably 80 m²/g or more, particularly preferably 100 m²/g or more, and is 300 m²/g or less, preferably 250 m²/g or less, more preferably 200 m²/g or less. The inorganic filler (B) may have a specific surface area of 190 m²/g or less, 170 m²/g or less, or 150 m²/g or less. A further improvement of polishability can be attained by setting these lower limits for the specific surface area of inorganic filler (B). By setting the foregoing upper limits for the specific surface area of inorganic filler (B), the dental composition can contain increased amounts of inorganic filler (B), and the mechanical strength of the cured product improves. When the inorganic filler (B) is an agglomerated particle (secondary particle), the specific surface area of inorganic filler (B) means a specific surface area of the agglomerated particle (secondary particle).

The specific surface area of inorganic filler (B) can be determined using a BET method. Specifically, the specific surface area of inorganic filler (B) can be measured using, for example, a specific surface area measurement device (for example, BELSORP-mini series manufactured by MicrotracBEL Corp.). For the specific surface area measurement of inorganic filler (B) using such a method, it is preferable that the specific surface area be measured after ashing the inorganic filler (B), for example, by treating the inorganic filler (B) at 450°C for 4 hours, in order to eliminate the influence of a surface treatment when the inorganic filler (B) is subjected to a surface treatment, as will be described later. Preferably, ashing is performed for the specific surface area measurement of the inorganic filler (B) contained in the dental composition. The specific surface area of inorganic filler (B) can be measured using the method specifically described in the EXAMPLES section below.

The overall particle shape of inorganic filler (B) is not particularly limited, and the inorganic filler (B) may be used in the form of an irregularly shaped powder or a spherical powder. An irregularly shaped inorganic filler (B) improves particularly the mechanical strength and wear resistance of the cured product obtained. With a spherical inorganic filler (B), the dental composition can have improved ease of handling with smooth and readily spreadable paste properties. The overall shape of inorganic filler (B) can be appropriately selected according to the intended use of the dental composition.

The refractive index of inorganic filler (B) is not particularly limited. However, the transparency of the cured product can easily increase when the inorganic filler (B) has a refractive index close to the refractive indices of the components of dental composition other than the inorganic filler (B). In this respect, the refractive index of inorganic filler (B) is preferably 1.45 or more, more preferably 1.50 or more, even more preferably 1.51 or more, and is preferably 1.63 or less, more preferably 1.60 or less, even more preferably 1.58 or less. The refractive index of inorganic filler (B) can be controlled by, for example, adjusting the types and proportions of metallic elements contained in the inorganic filler (B).

The material of inorganic filler (B) is not particularly limited. For advantages such as enhancement of the effectiveness of the present invention, it is preferable that the inorganic filler (B) comprise silicon dioxide, and at least one other metal oxide (metal oxide other than silicon dioxide). Examples of such other metal oxides include zirconium oxide, titanium oxide, barium oxide, lanthanum oxide, ytterbium oxide, strontium oxide, hafnium oxide, zinc oxide, boron oxide, and aluminum oxide. These other metal oxides may be used alone, or two or more thereof may be used in combination. In view of radiopacity, refractive index, and availability, the other metal oxides are preferably at least one selected from the group consisting of zirconium oxide, barium oxide, lanthanum oxide, and ytterbium oxide.

The silicon dioxide content in the inorganic filler (B) is not particularly limited. However, the silicon dioxide content is preferably 5 mass% or more, more preferably 10 mass% or more, and is preferably 95 mass% or less, more preferably 90 mass% or less. By setting these lower limits for the silicon dioxide content, the refractive index difference between inorganic filler (B) and the constituent components of dental composition other than inorganic filler (B) can be made smaller to improve the transparency of the cured product obtained. By setting the foregoing upper limits for the silicon dioxide content, the inorganic filler (B) can impart sufficient radiopacity for the dental composition.

The content of other metal oxides in the inorganic filler (B) is not particularly limited either. However, the content of other metal oxides is preferably 5 mass% or more, more preferably 10 mass% or more, and is preferably 95 mass% or less, more preferably 90 mass% or less. By setting these lower limits for the content of other metal oxides, the inorganic filler (B) can impart sufficient radiopacity for the dental composition. By setting the foregoing upper limits for the content of other metal oxides, the refractive index difference between inorganic filler (B) and the constituent components of dental composition other than inorganic filler (B) can be made smaller to improve the transparency of the cured product obtained.

The inorganic filler (B) may be amorphous or crystalline, or may be an amorphous-crystalline mixture. Preferably, the inorganic filler (B) contains at least an amorphous component. Transparency improves with increasing fraction of the amorphous component in the inorganic filler (B).

The structure of inorganic filler (B) is not particularly limited, and the inorganic filler (B) may have a structure with primary particles dispersed therein. For advantages such as enhancement of the effectiveness of the present invention, it is preferable that the inorganic filler (B) contain secondary particles (particularly, agglomerated particles or the like) formed by binding of inorganic primary fine particles. Typically, such secondary particles are formed by inorganic primary fine particles partly binding to one another.

The average particle diameter (average primary particle diameter) of the inorganic primary fine particles forming secondary particles is not particularly limited. However, the average particle diameter of inorganic primary fine particles is preferably 2 nm or more, more preferably 5 nm or more, and is preferably 300 nm or less, more preferably 200 nm or less because an overly small average particle diameter tends to cause decrease of the mechanical strength of the cured product whereas an overly large average particle diameter may lead to decrease of polishability. The average particle diameter of inorganic primary fine particles can be determined using a scanning electron microscope. Specifically, for example, the average particle diameter can be determined by taking a micrograph using an electron microscope such as an SU3500 or SU9000 manufactured by Hitachi, Ltd., and finding a mean value of particle diameters of randomly selected 20 particles. For non-spherical particles, the particle diameter can be determined by calculating an arithmetic mean value of the maximum and minimum lengths of particles.

In an inorganic filler (B) containing silicon dioxide and at least one other metal oxide and in which inorganic primary fine particles are forming secondary particles by binding to one another, particles of primarily silicon dioxide and particles of primarily other metal oxide may independently exist as inorganic primary fine particles, and the secondary particles may be particles formed by binding of these two components. Alternatively, secondary particles may be particles formed by binding of inorganic primary fine particles containing silicon dioxide and other metal oxide. In the case of the latter, the inorganic primary fine particles may be in the form of a solid solution of silicon dioxide and other metal oxide, or may have a structure in which a coating of primarily other metal oxide is covering particles of primarily silicon dioxide, or in which a coating of primarily silicon dioxide is covering particles of primarily other metal oxide. The secondary particles formed by binding of inorganic primary fine particles containing silicon dioxide and other metal oxide may also be a combination of these different forms.

The inorganic primary fine particles forming secondary particles may be, for example, particles having the foregoing average particle diameter (average primary particle diameter) as a result of pulverization of particles commonly used as composite material in dentistry, such as silicon dioxide particles, particles of other metal oxide, and particles containing silicon dioxide and other metal oxide. As another example, the inorganic primary fine particles forming secondary particles may be spherical particles synthesized by using the sol-gel method, or ultrafine silica particles obtained by spray pyrolysis, or may be commercially available particles.

Inorganic primary fine particles having a structure in which a coating of primarily other metal oxide is covering particles of primarily silicon dioxide can be obtained by, for example, using the method described in Patent Literature 1. Specifically, an aqueous solution of metal salt capable of forming other metal oxide is added into silica sol under stirring, and, through hydrolysis and other reactions, inorganic primary fine particles can be formed that have a structure in which the metal oxide component is covering the silica sol.

The method of production of the inorganic filler (B) used for a dental composition of the present invention is not particularly limited, and the inorganic filler (B) may be one obtained by using any method. Specifically, in the case of an inorganic filler (B) containing silicon dioxide and at least one other metal oxide and in which inorganic primary fine particles are forming secondary particles by binding to one another, it is preferable for advantages such as easier production of inorganic filler (B) that the inorganic filler (B) be produced by forming an aggregate of inorganic primary fine particles from primary particles such as silicon dioxide particles, particles of other metal oxide, or particles containing silicon dioxide and other metal oxide, and weakly fusing and binding the inorganic primary fine particles to one another in the aggregate in a heat treatment.

The procedures for forming an aggregate of inorganic primary fine particles are not particularly limited, and may be appropriately selected from methods that can produce aggregates of desired sizes from inorganic primary fine particles. In a convenient method of forming an aggregate, the inorganic primary fine particles are dispersed in a dispersion medium, and the dispersion medium is removed from the dispersion using a method such as heating or vacuuming. Another convenient method is a method generally called spray drying, in which a dispersion of inorganic primary fine particles is sprayed in fine droplets into a drying chamber to obtain a dry aggregate. The former method may be followed by procedures such as crushing and pulverization, as needed, because the method may result in producing aggregates forming excessively large clumps. Spray drying is more efficient because this step can be omitted in many cases. When the cohesion holding the inorganic primary fine particles in the aggregate is weak, and the aggregate cannot easily retain its form, a binder may be used that is commonly used as an auxiliary agent for molding of ceramic raw material (for example, a water-soluble polymer compound, such as polyvinyl alcohol), irrespective of the procedure used to form an aggregate.

The heat treatment conditions for aggregate cannot be generalized as a rule because the optimum conditions (temperature, time) depend on variables such as the composition of the inorganic primary fine particles. For many compositions, however, the preferred heat treatment temperature (firing temperature) ranges from 500 to 1,200°C. An overly low heat treatment temperature tends to cause decrease of the mechanical strength of the cured product of the dental composition finally obtained. An overly high heat treatment temperature causes excessive fusing between inorganic primary fine particles, and this tends to impair the polishability and gloss retention of the cured product obtained from the final dental composition.

More detailed conditions for the heat treatment of aggregate can be determined as follows. For example, the inorganic primary fine particles are fired under different conditions in the foregoing heat treatment temperature ranges to form different inorganic fillers (B) as secondary particles (agglomerated particles), and these particles are analyzed by powder X-ray diffraction analysis. The conditions that produced inorganic fillers (B) with no observable crystalline structure can then be decided as the heat treatment conditions for aggregate. As another example, dental compositions produced from inorganic fillers (B) formed in this fashion may be formed into cured products, and the heat treatment conditions may be decided by examining properties such as the flexural strength of the cured products, or the gloss on polished surfaces of the cured products. In many cases, an insufficient heat treatment leads to a cured product with insufficient flexural strength. When overly heat treated, the resultant cured product tends to have a low gloss on polished surfaces, in addition to having an unnaturally opaque appearance. This is probably a result of crystallization occurring in parts of the constituent components, increasing the refractive index of the inorganic filler (B) and producing an unnaturally white color, different from the whiteness of natural teeth, in a cured product formed by the dental composition using such an inorganic filler (B). The undesirable effect of over heat treatment also can be explained by increased hardness of inorganic filler (B), making it difficult to grind a cured product formed by the dental composition, and impairing polishability.

The product of firing by the heat treatment of the aggregate may be used as an inorganic filler (B) as it is, or may be used after optional particle size adjustments by being pulverized using a mortar, a ball mill, or the like.

A surface treatment is not necessarily required for the inorganic filler (B). However, a surface treatment is preferred for a number of advantages, including improved compatibility with the polymerizable monomer (A) by making the surface of inorganic filler (B) hydrophobic, and increased amounts of inorganic filler (B). A surface treatment may be carried out using a surface treatment agent. The type of surface treatment agent is not particularly limited, and known surface treatment agents may be used. For example, the surface treatment agent may be a silane coupling agent, an organotitanium coupling agent, an organozirconium coupling agent, or an organoaluminum coupling agent. The surface treatment agent may be used alone, or two or more thereof may be used in combination. In view of availability and properties such as compatibility between polymerizable monomer (A) and inorganic filler (B), preferred are silane coupling agents.

The silane coupling agent is not particularly limited. However, the silane coupling agent is preferably a compound represented by the following general formula (3).

H₂C=CR³-CO-R⁴-(CH₂)_{q}-SiR⁵ₚR⁶₍₃₋ₚ₎ (3),

wherein R³ is a hydrogen atom or a methyl group, R⁴ is an oxygen atom, a sulfur atom, or -NR⁷-, where R⁷ is a hydrogen atom or an aliphatic group having 1 to 8 carbon atoms (may be linear, branched, or cyclic), R⁵ is a hydrolyzable group, R⁶ is a hydrocarbon group having 1 to 6 carbon atoms, p is an integer of 1 to 3, and q is an integer of 1 to 13, where a plurality of R⁵ and R⁶ each may be the same or different from each other.

In the general formula (3), R³ is a hydrogen atom or a methyl group, preferably a methyl group. R⁴ is an oxygen atom, a sulfur atom, or -NR⁷-, preferably an oxygen atom. R⁷ is a hydrogen atom or an aliphatic group having 1 to 8 carbon atoms (may be linear, branched, or cyclic), and the aliphatic group having 1 to 8 carbon atoms represented by R⁷ may be a saturated aliphatic group (e.g., an alkyl group, or a cycloalkylene group such as cyclohexyl) or an unsaturated aliphatic group (e.g., an alkenyl group, an alkynyl group). In view of availability and properties such as ease of production and chemical stability, the aliphatic group having 1 to 8 carbon atoms represented by R⁷ is preferably a saturated aliphatic group, more preferably an alkyl group. Examples of the alkyl group include methyl, ethyl, n-propyl, isopropyl, n-butyl, isobutyl, sec-butyl, tert-butyl, n-pentyl, isopentyl, n-hexyl, n-heptyl, 2-methylhexyl, and n-octyl. R⁷ is preferably a hydrogen atom or an alkyl group having 1 to 4 carbon atoms, more preferably a hydrogen atom or an alkyl group having 1 to 3 carbon atoms, even more preferably a hydrogen atom.

Examples of the hydrolyzable group represented by R⁵ in the general formula (3) include alkoxy groups such as methoxy, ethoxy, and butoxy; halogen atoms such as a chlorine atom and a bromine atom; and an isocyanate group. When a plurality of R⁵ exists, the plurality of R⁵ may be the same or different from each other. R⁵ is preferably an alkoxy group, more preferably methoxy or ethoxy, even more preferably methoxy.

Examples of the hydrocarbon group having 1 to 6 carbon atoms represented in R⁶ in the general formula (3) include an alkyl group having 1 to 6 carbon atoms (may be cyclic), an alkenyl group having 2 to 6 carbon atoms (may be cyclic), and an alkynyl group having 2 to 6 carbon atoms. When a plurality of R⁶ exists, the plurality of R⁶ may be the same or different from each other.

Examples of the alkyl group having 1 to 6 carbon atoms include methyl, ethyl, n-propyl, isopropyl, n-butyl, isobutyl, sec-butyl, tert-butyl, n-pentyl, isopentyl, neopentyl, tert-pentyl, n-hexyl, cyclopropyl, cyclobutyl, cyclopentyl, and cyclohexyl.

Examples of the alkenyl group having 2 to 6 carbon atoms include vinyl, allyl, 1-methylvinyl, 1-propenyl, butenyl, pentenyl, hexenyl, cyclopropenyl, cyclobutenyl, cyclopentenyl, and cyclohexenyl.

Examples of the alkynyl group having 2 to 6 carbon atoms include ethynyl, 1-propynyl, 2-propynyl, 1-butynyl, 1-methyl-2-propynyl, 2-butynyl, 3-butynyl, 1-pentynyl, 1-ethyl-2-propynyl, 2-pentynyl, 3-pentynyl, 1-methyl-2-butynyl, 4-pentynyl, 1-methyl-3-butynyl, 2-methyl-3-butynyl, 1-hexynyl, 2-hexynyl, 1-ethyl-2-butynyl, 3-hexynyl, 1-methyl-2-pentynyl, 1-methyl-3-pentynyl, 4-methyl-1-pentynyl, 3-methyl-1-pentynyl, 5-hexynyl, and 1-ethyl-3-butynyl.

In the general formula (3), p is an integer of 1 to 3, preferably 2 or 3, more preferably 3. In the general formula (3), q is an integer of 1 to 13, preferably an integer of 2 to 12, more preferably an integer of 3 to 11.

Specific examples of the silane coupling agent represented by the general formula (3) include methacryloyloxymethyltrimethoxysilane, 2-methacryloyloxyethyltrimethoxysilane, 3-methacryloyloxypropyltrimethoxysilane, 4-methacryloyloxybutyltrimethoxysilane, 5-methacryloyloxypentyltrimethoxysilane, 6-methacryloyloxyhexyltrimethoxysilane, 7-methacryloyloxyheptyltrimethoxysilane, 8-methacryloyloxyoctyltrimethoxysilane, 9-methacryloyloxynonyltrimethoxysilane, 10-methacryloyloxydecyltrimethoxy silane, 11-methacryloyloxyundecyltrimethoxysilane, 12 -methacryloyloxydodecyltrimethoxysilane, 13-methacryloyloxytridecyltrimethoxysilane, 11-methacryloyloxyundecyldichloromethylsilane, 11-methacryloyloxyundecyltrichlorosilane, and 12-methacryloyloxydodecyldimethoxymethylsilane. The silane coupling agent may be used alone, or two or more thereof may be used in combination. In view of availability, 3-methacryloyloxypropyltrimethoxysilane is preferred. In view of improved compatibility between polymerizable monomer (A) and inorganic filler (B), 8-methacryloyloxyoctyltrimethoxysilane, 9-methacryloyloxynonyltrimethoxysilane, 10-methacryloyloxydecyltrimethoxysilane, and 11-methacryloyloxyundecyltrimethoxysilane are preferred.

The surface treatment method is not particularly limited, and a known method may be used.

The amount of surface treatment agent used is not particularly limited, and may be, for example, at least 1 part by mass relative to 100 parts by mass of the inorganic filler before surface treatment. The amount of surface treatment agent is preferably at least 5 parts by mass, more preferably at least 6 parts by mass, even more preferably at least 8 parts by mass, particularly preferably at least 10 parts by mass, most preferably at least 20 parts by mass, and is preferably at most 50 parts by mass, more preferably at most 45 parts by mass, even more preferably at most 40 parts by mass. By setting these lower limits for the amount of surface treatment agent, the mechanical strength of the cured product can more easily improve. By setting the foregoing upper limits for the amount of surface treatment agent, a decrease of the mechanical strength of the cured product due to the excessive surface treatment agent can be reduced.

As a rule, a surface treatment of an inorganic filler with a surface treatment agent hydrophobizes the surface of inorganic filler and improves the compatibility with a polymerizable monomer. This allows the composition to contain increased amounts of inorganic filler. In an inorganic filler (B) having an average particle diameter of 1 to 20 µm and a specific surface area of 10 to 300 m²/g as in the present invention, a surface treatment with a relatively large amount of surface treatment agent more effectively improves the mechanical strength and other properties of the cured product obtained.

The content of inorganic filler (B) in a dental composition of the present invention (the content of inorganic filler (B) after surface treatment when subjected to a surface treatment) is not particularly limited. However, in view of properties such as ease of handling of the dental composition or the mechanical strength of the cured product obtained, the content of inorganic filler (B) is preferably 10 mass% or more, more preferably 30 mass% or more, even more preferably 50 mass% or more, particularly preferably 65 mass% or more relative to the mass of the dental composition, and is preferably 97 mass% or less, more preferably 95 mass% or less, even more preferably 90 mass% or less relative to the mass of the dental composition. By setting these lower limits for the content of inorganic filler (B), the cured product can have improved mechanical strength, and the dental composition can have improved ease of handling because of effectively reduced stickiness. By setting the foregoing upper limits for the content of inorganic filler (B), the dental composition can be prevented from becoming too hard, and ease of handling improves. The inorganic filler (B) may be used alone, or two or more thereof may be used in combination.

### Prepolymer (C)

### Properties and Structure of Prepolymer (C)

A dental composition of the present invention comprises a prepolymer (C). In the present invention, "prepolymer" refers to an intermediate of a polymerization reaction of a polymerizable monomer after the reaction is stopped at an appropriate point, or a polymer having a functional group introduced therein after polymerization. In either case, the prepolymer has an unreacted polymerizable functional group that enables further polymerization (for example, polymerization with polymerizable monomer (A)). The prepolymer (C) may be used alone, or two or more thereof may be used in combination.

The unreacted polymerizable functional group of prepolymer (C) is not particularly limited. Examples of the unreacted polymerizable functional group include a carbon-carbon double bond, a vinyl group, a vinyloxy group, a (meth)allyl group, a (meth)acryloyl group, a maleoyl group, a styryl group, and a cinnamoyl group. The unreacted polymerizable functional group is preferably a (meth)acryloyl group, more preferably a (meth)acryloyloxy group or a (meth)acrylamide group. The prepolymer (C) has, on average, preferably at least 1, more preferably at least 2, even more preferably at least 5, particularly preferably at least 10 unreacted polymerizable functional groups per molecule. The prepolymer (C) may have, on average, at least 15, at least 20, or at least 25 unreacted polymerizable functional groups per molecule. The prepolymer (C) has, on average, preferably at most 1,000, more preferably at most 500, even more preferably at most 100, particularly preferably at most 50 unreacted polymerizable functional groups per molecule. The method used to measure the number of unreacted polymerizable functional groups in prepolymer (C) is not particularly limited. For example, the number of unreacted polymerizable functional groups can be determined by measuring the concentration (mol/g) of unreacted polymerizable functional groups in the prepolymer by NMR analysis, and multiplying the measured concentration by the weight-average molecular weight of prepolymer (C), as will be described later. More specifically, the number of unreacted polymerizable functional groups can be determined by using the method described in the EXAMPLES section below.

The molecular weight of prepolymer (C) is not particularly limited. For advantages such as enhancement of the effectiveness of the present invention, the weight-average molecular weight of prepolymer (C) is preferably 1,000 or more, more preferably 5,000 or more, even more preferably 10,000 or more, and is preferably 1,000,000 or less, more preferably 500,000 or less, even more preferably 300,000 or less, particularly preferably 100,000 or less. The weight-average molecular weight of prepolymer (C) may be 80,000 or less, or 60,000 or less. The method used to measure the weight-average molecular weight of prepolymer (C) is not particularly limited. For example, the weight-average molecular weight of prepolymer (C) can be measured by GPC, more specifically, the method described in the EXAMPLES section below.

### Polyfunctional Monomer (a)

Preferably, the prepolymer (C) has a structure derived from a polyfunctional monomer (a). By having a structure derived from a polyfunctional monomer (a), the prepolymer (C) is able to take a mesh-like multi-branched structure, and can increase its strength. This makes it possible to achieve an even higher mechanical strength in a cured product produced from a dental composition containing such a prepolymer (C). A prepolymer (C) having a mesh-like multi-branched structure can more easily take a spherical shape or a shape close to a sphere as a whole, and a dental composition containing such a prepolymer (C) does not experience as high a viscosity increase as a dental composition containing a common polymer. That is, a dental composition containing a prepolymer (C) having a structure derived from a polyfunctional monomer (a) can contain an increased amount of prepolymer (C) without causing a large change in the filling rate of inorganic filler (B) or in the ease of handling of the dental composition, and the polymerization shrinkage stress can be reduced by the increased fraction of prepolymer (C).

The molecular weight of polyfunctional monomer (a) is preferably 250 or more, more preferably 300 or more, even more preferably 400 or more, and is preferably 1,000 or less, more preferably 800 or less, even more preferably 700 or less. In a dental composition of the present invention, the mechanical strength improves probably as a result of the polymerizable monomer (A) penetrating into the prepolymer (C), and the prepolymer (C) merging with the matrix formed by the cured product of polymerizable monomer (A) upon polymerization and cure. With the foregoing lower limits set for the molecular weight of polyfunctional monomer (a), the prepolymer (C) is able to have a larger mesh-like multi-branched structure, allowing the polymerizable monomer (A) to penetrate more easily, and possibly improving the mechanical strength even further. By setting the foregoing upper limits for the molecular weight of polyfunctional monomer (a), a prepolymer (C) solution can have a reduced viscosity, and the dental composition can have improved ease of handling.

The polyfunctional monomer (a) may be a known polyfunctional monomer having two or more polymerizable functional groups per molecule, particularly preferably a polyfunctional monomer having two or more radical polymerizable functional groups. Examples of the polyfunctional monomer having two or more radical polymerizable functional groups include esters of unsaturated carboxylic acids such as α-cyanoacrylic acid, (meth)acrylic acid, α-halogenated acrylic acid, crotonic acid, cinnamic acid, sorbic acid, maleic acid, and itaconic acid; derivatives of (meth)acrylamide; vinyl esters; vinyl ethers; mono-N-vinyl derivatives; and derivatives of styrene. The prepolymer (C) may have a structure derived from one kind of polyfunctional monomer (a), or a structure derived from two or more kinds of polyfunctional monomers (a). In view of reactivity, it is preferable that the polyfunctional monomer (a) comprise at least one selected from the group consisting of an ester of an unsaturated carboxylic acid, and a derivative of (meth)acrylamide, more preferably at least one selected from the group consisting of a (meth)acrylic acid ester (a polyfunctional (meth)acrylic acid ester) and a derivative of (meth)acrylamide (a polyfunctional (meth)acrylamide), even more preferably a (meth)acrylic acid ester. In view of availability and advantages such as enhancement of the effectiveness of the present invention, the polyfunctional monomer (a) preferably comprises a compound represented by the following general formula (1).

X-A-X (1),

where X is a (meth)acryloyloxy group or a (meth)acrylamide group, and A is an optionally substituted divalent aliphatic hydrocarbon group having 5 or more carbon atoms, or an optionally substituted divalent aromatic hydrocarbon group having 6 or more carbon atoms, wherein some of the constituent carbon atoms in A may be substituted with oxygen atoms and/or nitrogen atoms, and a plurality of X may be the same or different from each other.

In the general formula (1), X is a (meth)acryloyloxy group or a (meth)acrylamide group, preferably a (meth)acryloyloxy group. In the general formula (1), a plurality of X may be the same or different from each other. Preferably, a plurality of X is the same.

In view of ease of handling of the dental composition and mechanical strength of the cured product, the divalent aliphatic hydrocarbon group represented by A in the general formula (1) has 5 or more carbon atoms, preferably 8 or more carbon atoms, more preferably 10 or more carbon atoms, and has preferably 150 or less carbon atoms, more preferably 60 or less carbon atoms, even more preferably 50 or less carbon atoms (before substitution with oxygen atoms and/or nitrogen atoms when some of the carbon atoms are substituted with these atoms).

The divalent aliphatic hydrocarbon group represented by A may be linear, branched, or cyclic. Examples of the divalent aliphatic hydrocarbon group include linear or branched alkylene groups such as various types of pentylene groups, hexylene groups, heptylene groups, octylene groups, nonylene groups, decylene groups, undecylene groups, dodecylene groups, tridecylene groups, tetradecylene groups, pentadecylene groups, hexadecylene groups, heptadecylene groups, octadecylene groups, nonadecylene groups, and eicosylene groups; and cycloalkylene groups such as a cyclopropylene group, a cyclopentylene group, a cyclohexylene group, a cycloheptylene group, a cyclooctylene group, a decahydronaphthalenediyl group, a norbornanediyl group, an adamantanediyl group, and a tricyclo[5.2.1.0^{2,6}]decanediyl group. Aside from the alkylene groups and cycloalkylene groups, the divalent aliphatic hydrocarbon group represented by A may be an aliphatic hydrocarbon group having an unsaturated bond. In the divalent aliphatic hydrocarbon group represented by A, some of the constituent carbon atoms may be substituted with oxygen atoms (-O-) and/or nitrogen atoms (e.g., -NH-, =N-).

In view of ease of handling of the dental composition and mechanical strength of the cured product, the divalent aromatic hydrocarbon group represented by A in the general formula (1) has 6 or more carbon atoms, preferably 8 or more carbon atoms, more preferably 10 or more carbon atoms, and has preferably 150 or less carbon atoms, more preferably 60 or less carbon atoms, even more preferably 50 or less carbon atoms (before substitution with oxygen atoms and/or nitrogen atoms when some of the carbon atoms are substituted with these atoms).

The divalent aromatic hydrocarbon group represented by A is a divalent group having at least one aromatic ring, and may be a divalent group consisting of one or more aromatic rings, or may be a divalent group formed by one or more aromatic rings and one or more divalent aliphatic hydrocarbon groups. Examples of the latter include a group having a structure in which a plurality of aromatic rings bind one another via a divalent aliphatic hydrocarbon group having 1 to 30 carbon atoms, and a group having a C1 to C30 divalent aliphatic hydrocarbon group in portions attached to one of the Xs or both Xs.

Examples of groups forming the aromatic ring include arylene groups such as a phenylene group, a biphenylylene group, a terphenylylene group, a naphthylene group, an anthrylene group, a phenanthrylene group, and a pyrenylene group; and divalent heteroaromatic ring groups such as a pyridinylene group, a pyrimidinylene group, a furanylene group, a pyrrolylene group, a thiophenylene group, a quinolinylene group, a benzofuranylene group, a benzothiophenylene group, an indolylene group, a carbazolylene group, a benzooxazolylene group, a quinoxalinylene group, a benzoimidazolylene group, a pyrazolylene group, a dibenzofuranylene group, and a dibenzothiophenylene group.

In the divalent aromatic hydrocarbon group represented by A, some of the constituent carbon atoms may be substituted with oxygen atoms (-O-) and/or nitrogen atoms (e.g., -NH-, =N-).

The divalent aliphatic hydrocarbon group having 5 or more carbon atoms and divalent aromatic hydrocarbon group having 6 or more carbon atoms each may or may not have one or more optional substituents. Examples of such substituents include an alkoxy group having 1 to 5 carbon atoms, a halogen atom (a fluorine atom, a chlorine atom, a bromine atom, an iodine atom), a hydroxyl group, a carboxyl group, an amino group, and an acyl group. When substituents are present, the divalent aliphatic hydrocarbon group having 5 or more carbon atoms and divalent aromatic hydrocarbon group having 6 or more carbon atoms may have 1 to 30 substituents, preferably 1 to 10 substituents, more preferably 1 to 5 substituents, though the number of substituents is not particularly limited.

In view of availability and enhanced effectiveness of the present invention, it is particularly preferable that the polyfunctional monomer (a) comprise a compound represented by the following general formula (2).

X-(R¹)ₘ-A'-(R²)ₙ-X (2),

where X is a (meth)acryloyloxy group or a (meth)acrylamide group, R¹ and R² are each independently an optionally substituted alkyleneoxy group having 1 to 6 carbon atoms, A' is an optionally substituted divalent aliphatic hydrocarbon group having 3 or more carbon atoms, or an optionally substituted divalent aromatic hydrocarbon group having 6 or more carbon atoms, wherein some of the constituent carbon atoms in A' may be substituted with oxygen atoms and/or nitrogen atoms, m and n are each independently an integer of 1 or more, the average number of moles of alkyleneoxy groups added as represented by an average of the sum of m and n per molecule is 2 to 35, and a plurality of X, R¹, and R² each may be the same or different from each other.

In the general formula (2), X is a (meth)acryloyloxy group or a (meth)acrylamide group, preferably a (meth)acryloyloxy group. In the general formula (2), a plurality of X may be the same or different from each other. Preferably, a plurality of X is the same.

Examples of the alkyleneoxy groups having 1 to 6 carbon atoms represented by R¹ and R² in the general formula (2) include -CH₂-O-*, -(CH₂)₂-O-*, -(CH₂)₃-O-*, and -CH(CH₃)-CH₂-O-*. Preferably, the alkyleneoxy groups having 1 to 6 carbon atoms represented by R¹ and R² are -(CH₂)₂-O-*. Here, * indicates the side flanking A'. When a plurality of R¹ and/or R² exists in the general formula (2) (when m and/or n are integers of 2 or more), the plurality of R¹ and R² each may be the same or different from each other. Preferably, a plurality of R¹ is the same, and a plurality of R² is the same. R¹ and R² may be the same or different, and are preferably the same.

The alkyleneoxy group having 1 to 6 carbon atoms may or may not have one or more optional substituents. Examples of such substituents include an alkoxy group having 1 to 5 carbon atoms, a halogen atom (a fluorine atom, a chlorine atom, a bromine atom, an iodine atom), a hydroxyl group, a carboxyl group, an amino group, and an acyl group. When substituents are present, the alkyleneoxy group having 1 to 6 carbon atoms may have 1 to 30 substituents, preferably 1 to 10 substituents, more preferably 1 to 5 substituents, though the number of substituents is not particularly limited.

In the general formula (2), m and n are each independently an integer of 1 or more. The average number of moles of alkyleneoxy groups added (for example, the average number of moles of ethyleneoxy groups added) as represented by an average of the sum of m and n per molecule of the compound represented by the general formula (2) is 2 to 35. In view of availability and advantages such as enhancement of the effectiveness of the present invention, the average number of moles of alkyleneoxy groups added is preferably 2 to 20, more preferably 2 to 10.

In the general formula (2), the divalent aliphatic hydrocarbon group represented by A' has 3 or more carbon atoms, preferably 4 or more carbon atoms, more preferably 5 or more carbon atoms, and has preferably 30 or less carbon atoms, more preferably 20 or less carbon atoms (before substitution with oxygen atoms and/or nitrogen atoms when some of the carbon atoms are substituted with these atoms).

The divalent aliphatic hydrocarbon group represented by A' may be linear, branched, or cyclic. Examples of the divalent aliphatic hydrocarbon group represented by A' include various types of propylene groups, butylene groups, and the linear or branched alkylene or cycloalkylene group exemplified above for the divalent aliphatic hydrocarbon group represented by A in conjunction with the descriptions of general formula (1). Aside from the alkylene groups and cycloalkylene groups, the divalent aliphatic hydrocarbon group represented by A' may be an aliphatic hydrocarbon group having an unsaturated bond. Some of the constituent carbon atoms in the divalent aliphatic hydrocarbon group represented by A' may be substituted with oxygen atoms (-O-) and/or nitrogen atoms (e.g., -NH-, =N-).

In view of availability and the mechanical strength of the cured product obtained, the divalent aliphatic hydrocarbon group represented by A' is preferably a group represented by any of the following formulae (A'-1-1) to (A'-1-6). In the following formulae (A'-1-1) to (A'-1-6), Z represents a single bond, -C(=O)-, or -NH-C(=O)-, and a plurality of Z may be the same or different from each other.

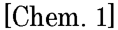

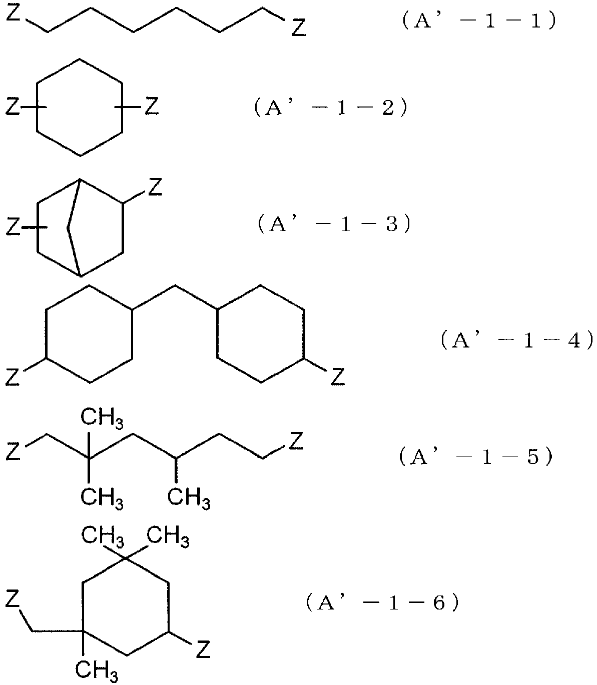

In the general formula (2), the divalent aromatic hydrocarbon group represented by A' has 6 or more carbon atoms, preferably 8 or more carbon atoms, more preferably 10 or more carbon atoms, and has preferably 30 or less carbon atoms, more preferably 20 or less carbon atoms (before substitution with oxygen atoms and/or nitrogen atoms when some of the carbon atoms are substituted with these atoms).

Examples of the divalent aromatic hydrocarbon group represented by A' include the aromatic hydrocarbon groups exemplified above for the divalent aromatic hydrocarbon group represented by A in conjunction with the descriptions of general formula (1). Some of the constituent carbon atoms in the divalent aromatic hydrocarbon group represented by A' may be substituted with oxygen atoms (-O-) and/or nitrogen atoms (e.g., -NH-, =N-).

In view of availability and the mechanical strength of the cured product obtained, the divalent aromatic hydrocarbon group represented by A' is preferably a group represented by any of the following formulae (A'-2-1) to (A'-2-10). In the following formulae (A'-2-1) to (A'-2-10), k represents an integer of 1 to 20, Z represents a single bond, -C(=O)-, or -NH-C(=O)-, and a plurality of Z may be the same or different from each other.

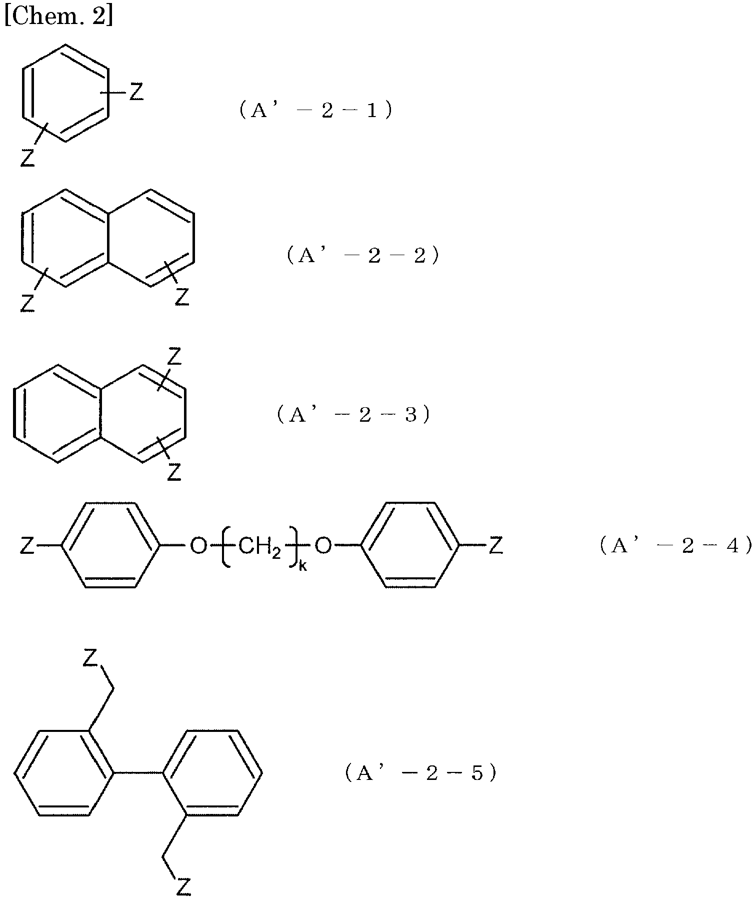

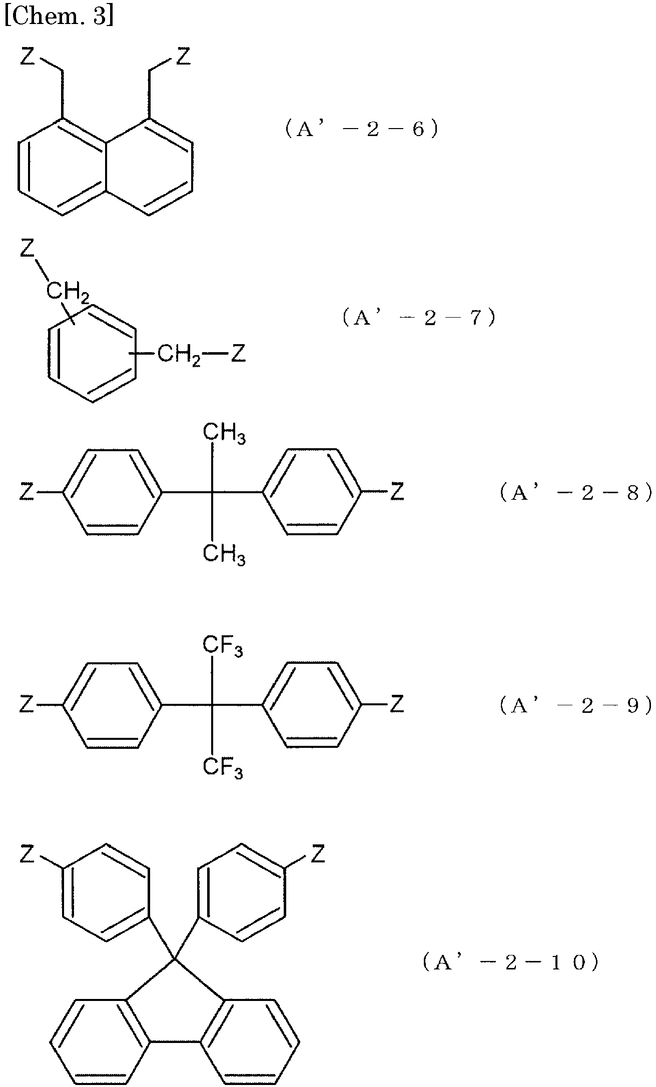

In the general formula (2), the divalent aliphatic hydrocarbon group having 3 or more carbon atoms and divalent aromatic hydrocarbon group having 6 or more carbon atoms represented by A' may or may not have one or more optional substituents. Examples of such substituents include an alkoxy group having 1 to 5 carbon atoms, a halogen atom (a fluorine atom, a chlorine atom, a bromine atom, an iodine atom), a hydroxyl group, a carboxyl group, an amino group, and an acyl group. When substituents are present, the divalent aliphatic hydrocarbon group having 3 or more carbon atoms and divalent aromatic hydrocarbon group having 6 or more carbon atoms may have 1 to 30 substituents, preferably 1 to 10 substituents, more preferably 1 to 5 substituents, though the number of substituents is not particularly limited.

In view of availability and the mechanical strength of the cured product obtained, the polyfunctional monomer (a) is preferably 1,2-bis(3-methacryloyloxy-2-hydroxypropyloxy)ethane, 2,2,4-trimethylhexamethylenebis(2-carbamoyloxyethyl)dimethacrylate (UDMA), 2,2-bis(4-methacryloyloxypolyethoxyphenyl)propane (average number of moles of ethyleneoxy groups added: 2 to 10), 2,2-bis[4-(3-methacryloyloxy-2-hydroxypropoxy)phenyl]propane (Bis-GMA), and 9,9-bis[4-(2-acryloyloxyethoxy)phenyl]fluorene.

### Monofunctional Monomer (b)

For advantages such as enhancement of the effectiveness of the present invention, it is preferable that the prepolymer (C) additionally have a structure derived from a monofunctional monomer (b). The monofunctional monomer (b) may be a known monofunctional monomer having one polymerizable functional group per molecule, preferably a monofunctional monomer having one radical polymerizable functional group. Examples of the monofunctional monomer having one radical polymerizable functional group include esters of unsaturated carboxylic acids such as α-cyanoacrylic acid, (meth)acrylic acid, α-halogenated acrylic acid, crotonic acid, cinnamic acid, sorbic acid, maleic acid, and itaconic acid; (meth)acrylamide; derivatives of (meth)acrylamide; vinyl esters; vinyl ethers; mono-N-vinyl derivatives; and derivatives of styrene. The prepolymer (C) may have a structure derived from one type of monofunctional monomer (b), or a structure derived from two or more types of monofunctional monomers (b). In view of reactivity, it is preferable that the monofunctional monomer (b) comprise at least one selected from the group consisting of an ester of an unsaturated carboxylic acid, and a derivative of (meth)acrylamide, more preferably at least one selected from the group consisting of a (meth)acrylic acid ester (a monofunctional (meth)acrylic acid ester), and a derivative of (meth)acrylamide (a monofunctional (meth)acrylamide), even more preferably a (meth)acrylic acid ester.

Specific examples of the monofunctional monomer (b) include:
alkyl (meth)acrylates such as methyl (meth)acrylate, ethyl (meth)acrylate, n-propyl (meth)acrylate, isopropyl (meth)acrylate, n-butyl (meth)acrylate, isobutyl (meth)acrylate, tert-butyl (meth)acrylate, n-pentyl (meth)acrylate, sec-amyl (meth)acrylate, isoamyl (meth)acrylate, hexyl (meth)acrylate, heptyl (meth)acrylate, n-octyl (meth)acrylate, isooctyl (meth)acrylate, 2-ethylhexyl (meth)acrylate, nonyl (meth)acrylate, n-decyl (meth)acrylate, isodecyl (meth)acrylate, undecyl (meth)acrylate, dodecyl (meth)acrylate, stearyl (meth)acrylate, and isostearyl (meth)acrylate;
fluoroalkyl (meth)acrylates such as trifluoroethyl (meth)acrylate, tetrafluoropropyl (meth)acrylate, hexafluoroisopropyl (meth)acrylate, octafluoropentyl (meth)acrylate, and heptadecafluorodecyl (meth)acrylate;
alkoxyalkyl (meth)acrylates such as methoxyethyl (meth)acrylate, ethoxyethyl (meth)acrylate, propoxyethyl (meth)acrylate, butoxyethyl (meth)acrylate, and methoxybutyl (meth)acrylate;
polyethylene glycol (meth)acrylates such as polyethylene glycol mono(meth)acrylate, ethoxydiethylene glycol (meth)acrylate, and methoxypolyethylene glycol (meth)acrylate;
polypropylene glycol (meth)acrylates such as polypropylene glycol mono(meth)acrylate, methoxypolypropylene glycol (meth)acrylate, and ethoxypolypropylene glycol (meth)acrylate;
alicyclic (meth)acrylates such as cyclohexyl (meth)acrylate, 4-butylcyclohexyl (meth)acrylate, dicyclopentanyl (meth)acrylate, dicyclopentenyl (meth)acrylate, dicyclopentadienyl (meth)acrylate, bornyl (meth)acrylate, isobornyl (meth)acrylate, and tricyclodecanyl (meth)acrylate;
hydroxyalkyl (meth)acrylates such as 2-hydroxyethyl (meth)acrylate, 3-hydroxypropyl (meth)acrylate, 4-hydroxybutyl (meth)acrylate, and 6-hydroxyhexyl (meth)acrylate;
aromatic (meth)acrylates such as benzyl (meth)acrylate, biphenylmethyl (meth)acrylate, phenoxyethylene glycol (meth)acrylate, phenoxydiethylene glycol (meth)acrylate, 3-phenoxybenzyl (meth)acrylate, nonylphenoxypolyethylene glycol (meth)acrylate, ethoxylated-o-phenylphenol (meth)acrylate, neopentyl glycol-(meth)acrylic acid-benzoic acid ester, and a quaternary salt of dimethylaminoethyl (meth)acrylate benzyl chloride;
derivatives of (meth)acrylamide such as N-methylol(meth)acrylamide, N-hydroxyethyl (meth)acrylamide, N,N-bis(hydroxyethyl)(meth)acrylamide, N,N-dimethyl (meth)acrylamide, N,N-diethyl (meth)acrylamide, N,N-di-n-propyl (meth)acrylamide, and N-ethyl-N-methyl (meth)acrylamide; and
functional group-containing (meth)acrylates such as 2-(N,N-dimethylamino)ethyl (meth)acrylate, 2,3-dibromopropyl (meth)acrylate, 3-(meth)acryloyloxypropyltrimethoxysilane, 11-(meth)acryloyloxyundecyltrimethoxysilane, (meth)acryloylmorpholine, (meth)acryloyloxydodecylpyridinium bromide, (meth)acryloyloxydodecylpyridinium chloride, (meth)acryloyloxyhexadecylpyridinium bromide, and (meth)acryloyloxyhexadecylpyridinium chloride.

In view of ease of handling of the dental composition, preferred are those having no hydroxyl group, more preferably alkyl (meth)acrylates, alicyclic (meth)acrylates, aromatic (meth)acrylates, even more preferably methyl (meth)acrylate, dodecyl (meth)acrylate, isobornyl (meth)acrylate, benzyl (meth)acrylate, phenoxyethylene glycol (meth)acrylate, and ethoxylated-o-phenylphenol (meth)acrylate, particularly preferably dodecyl (meth)acrylate, isobornyl (meth)acrylate, and phenoxyethylene glycol (meth)acrylate.

The prepolymer (C) has a refractive index of preferably 1.40 or more, more preferably 1.45 or more, even more preferably 1.50 or more, and a refractive index of preferably 1.70 or less, more preferably 1.65 or less, even more preferably 1.60 or less. With the refractive index of prepolymer (C) confined in these ranges, it becomes easier to reduce the refractive index difference from polymerizable monomer (A) and inorganic filler (B), and the dental composition produced can have improved transparency. The cure depth also improves in curing. With the foregoing refractive indices, it is also possible to further improve the mechanical strength of the cured product because the improved transparency of dental composition enables the polymerization reaction to sufficiently proceed even with a short photoirradiation time.

When the prepolymer (C) has both a structure derived from a polyfunctional monomer (a) and a structure derived from a monofunctional monomer (b), it is preferable that at least one of the polyfunctional monomer (a) and the monofunctional monomer (b) has an aromatic ring. Here, the aromatic ring includes an aromatic hydrocarbon ring and a heteroaromatic ring, and these may be monovalent or divalent, or may have higher valency. The refractive index of prepolymer (C) can be more easily adjusted to fall in the foregoing ranges when at least one of the polyfunctional monomer (a) and the monofunctional monomer (b) has an aromatic ring. With an aromatic ring present in the prepolymer (C), the polymer network after polymerization and cure can become more rigid, and the mechanical strength of the cured product improves even more.

The prepolymer (C) has a hydroxyl number of preferably 250 mgKOH/g or less, more preferably 230 mgKOH/g or less, even more preferably 200 mgKOH/g or less, particularly preferably 170 mgKOH/g or less, most preferably 100 mgKOH/g or less. With the hydroxyl number of prepolymer (C) confined in these ranges, hydrogen bond interactions with the polymerizable monomer (A) can be inhibited to reduce thickening of dental composition. This makes it possible to increase the content of inorganic filler (B), and the cured product obtained can have even higher mechanical strength. By confining the hydroxyl number of prepolymer (C) in the foregoing ranges, interactions with inorganic filler (B) can be inhibited to reduce decrease of the consistency of the dental composition, and the dental composition can have improved ease of handling. With the prepolymer (C) having a hydroxyl number in the foregoing ranges, it is also possible to produce the desired polymerization shrinkage stress, and improve the water resistance of the cured product obtained.

When the prepolymer (C) has both a structure derived from a polyfunctional monomer (a) and a structure derived from a monofunctional monomer (b), the mole ratio of the structure derived from a polyfunctional monomer (a) to the structure derived from a monofunctional monomer (b) is preferably 10/90 or more, more preferably 20/80 or more, even more preferably 30/70 or more, and is preferably 90/10 or less, more preferably 80/20 or less, even more preferably 70/30 or less. By setting these lower limits for the mole ratio, the crosslink density of the prepolymer (C) obtained improves, and the cured product can have even higher mechanical strength. By setting the foregoing upper limits for the mole ratio, the molecular weight of prepolymer (C) can be prevented from overly increasing, and the viscosity can be confined in the moderate range to improve ease of handling. The cured product can also have improved mechanical strength, and a further reduction of polymerization shrinkage stress can be achieved. It is also possible to inhibit gelation during the production of prepolymer (C).

Preferably, the prepolymer (C) has a structure derived from a chain transfer agent (c). Examples of the chain transfer agent (c) include:
thiol-based chain transfer agents such as thioglycerol, thioglycolic acid, 3-mercaptopropionic acid, thiomalic acid, 1-butanethiol, 1-octanethiol, 1-decanethiol, 3-decanethiol, 1-dodecanethiol, 1-octadecanethiol, cyclohexanethiol, thiophenol, octyl thioglycolate, octyl 2-mercaptopropionate, octyl 3-mercaptopropionate, 2-ethylhexyl mercaptopropionate, 2-mercaptoethanesulfonic acid, and 3-mercapto-2-butanol;
α-methylstyrene dimer-based chain transfer agents such as 2,4-diphenyl-4-methyl-1-pentene; and
hydrophilic chain transfer agents such as lower oxides, for example, phosphorous acid, hypophosphorous acid, sulfurous acid, hydrosulfurous acid, dithionous acid, and disulfurous acid, and salts thereof (for example, sodium hypophosphite, potassium hypophosphite, sodium sulfite, sodium bisulfite, sodium dithionite, sodium metabisulfite). Preferred are thiol-based chain transfer agents and α-methylstyrene dimer-based chain transfer agents, more preferably 1-octanethiol, 1-decanethiol, 3-mercapto-2-butanol, and
2,4-diphenyl-4-methyl-1-pentene. In view of toxicity, the chain transfer agent (c) is preferably not a compound having a molecular weight of 80 or less, such as 2-mercaptoethanol.

The content of prepolymer (C) in a dental composition of the present invention is not particularly limited. However, in view of consistency stability, polymerization shrinkage stress, and ease of handling of the dental composition, and the mechanical strength and other properties of the cured product obtained, the content of prepolymer (C) is preferably 0.5 mass% or more, more preferably 1 mass% or more, even more preferably 3 mass% or more relative to the mass of the dental composition, and is preferably 20 mass% or less, more preferably 18 mass% or less, even more preferably 16 mass% or less, and may be 12 mass% or less, or 8 mass% or less, relative to the mass of the dental composition.

### Method of Production of Prepolymer (C)

The method of production of prepolymer (C) is not particularly limited. However, the preferred method of production of prepolymer (C) is as follows, for example. Specifically, the prepolymer (C) can be produced by dissolving the polymerizable monomers (e.g., polyfunctional monomer (a), monofunctional monomer (b)), the chain transfer agent (c), and a polymerization initiator (d) in an organic solvent (e), and polymerizing the monomers.

When the polyfunctional monomer (a) and the monofunctional monomer (b) are both used as polymerizable monomers for the production of prepolymer (C), the mole ratio of polyfunctional monomer (a) to monofunctional monomer (b) is preferably 10/90 or more, more preferably 20/80 or more, even more preferably 30/70 or more, and is preferably 90/10 or less, more preferably 80/20 or less, even more preferably 70/30 or less because, as noted above, the cured product can have improved mechanical strength, and a sufficient reduction of polymerization shrinkage stress can be achieved when the mole ratio of polyfunctional monomer (a) to monofunctional monomer (b) is confined in these ranges.

The amount of chain transfer agent (c) used for the production of prepolymer (C) is preferably 5 to 120 mol relative to total 100 mol of the polyfunctional monomer (a) and monofunctional monomer (b). When the amount of chain transfer agent (c) is 5 mol or less, the molecular weight of prepolymer (C) may overly increase, and marked thickening of solution may occur. Using less than 5 mol of chain transfer agent (c) also may cause difficulties in production due to gelation occurring during production. With more than 120 mol of chain transfer agent (c), the prepolymer (C) produced may result in having a molecular weight that is too small, and the effect to reduce polymerization shrinkage stress may decrease. The more preferred amount of chain transfer agent (c) is 25 to 100 mol.

### Polymerization Initiator (d)

The polymerization initiator (d) used for the production of prepolymer (C) may be selected from polymerization initiators commonly used in industry, preferably those used in dentistry. Particularly preferred for use are photopolymerization initiators and chemical polymerization initiators. The polymerization initiator (d) may be used alone, or two or more thereof may be used in combination.

Examples of the photopolymerization initiators include (bis)acylphosphine oxides, ketals, α-diketones, and coumarin compounds.

Examples of acylphosphine oxides in the (bis)acylphosphine oxides include
2,4,6-trimethylbenzoyldiphenylphosphine oxide,
2,6-dimethoxybenzoyldiphenylphosphine oxide,
2,6-dichlorobenzoyldiphenylphosphine oxide,
2,4,6-trimethylbenzoylmethoxyphenylphosphine oxide,
2,4,6-trimethylbenzoylethoxyphenylphosphine oxide,
2,3,5,6-tetramethylbenzoyldiphenylphosphine oxide,
benzoyldi(2,6-dimethylphenyl)phosphonate, and salts thereof (for example, sodium salts, potassium salts, and ammonium salts). Examples of bisacylphosphine oxides include bis(2,6-dichlorobenzoyl)phenylphosphine oxide,
bis(2,6-dichlorobenzoyl)-2,5-dimethylphenylphosphine oxide,
bis(2,6-dichlorobenzoyl)-4-propylphenylphosphine oxide,
bis(2,6-dichlorobenzoyl)-1-naphthylphosphine oxide,
bis(2,6-dimethoxybenzoyl)phenylphosphine oxide,
bis(2,6-dimethoxybenzoyl)-2,4,4-trimethylpentylphosphine oxide,
bis(2,6-dimethoxybenzoyl)-2,5-dimethylphenylphosphine oxide,
bis(2,4,6-trimethylbenzoyl)phenylphosphine oxide,
bis(2,5,6-trimethylbenzoyl)-2,4,4-trimethylpentylphosphine oxide, and salts thereof (for example, sodium salts, potassium salts, and ammonium salts).

Particularly preferred among these (bis)acylphosphine oxides are
2,4,6-trimethylbenzoyldiphenylphosphine oxide,
2,4,6-trimethylbenzoylmethoxyphenylphosphine oxide,
bis(2,4,6-trimethylbenzoyl)phenylphosphine oxide, and sodium salts of 2,4,6-trimethylbenzoylphenylphosphine oxide.

Examples of the ketals include benzyl dimethyl ketal, and benzyl diethyl ketal.

Examples of the α-diketones include diacetyl, benzyl, camphorquinone, 2,3-pentadione, 2,3-octadione, 9,10-phenanthrenequinone, 4,4'-oxybenzyl, and acenaphthenequinone. Particularly preferred is camphorquinone for its maximum absorption wavelength occurring in the visible light region.

Examples of the coumarin compounds include the compounds mentioned in JP 9(1997)-3109 A and JP 10(1998)-245525 A, such as 3,3'-carbonyl bis(7-diethylaminocoumarin), 3-(4-methoxybenzoyl)coumarin, 3-thienyl coumarin, 3-benzoyl-5,7-dimethoxycoumarin, 3-benzoyl-7-methoxycoumarin, 3-benzoyl-6-methoxycoumarin, 3-benzoyl-8-methoxycoumarin, 3-benzoyl coumarin, 7-methoxy-3-(p-nitrobenzoyl)coumarin, 3-(p-nitrobenzoyl)coumarin, 3,5-carbonyl bis(7-methoxycoumarin), 3-benzoyl-6-bromocoumarin, 3,3'-carbonyl biscoumarin, 3-benzoyl-7-dimethylaminocoumarin, 3-benzoylbenzo[f]coumarin, 3-carboxycoumarin, 3-carboxy-7-methoxycoumarin, 3-ethoxycarbonyl-6-methoxycoumarin, 3-ethoxycarbonyl-8-methoxycoumarin, 3-acetylbenzo[f]coumarin, 3-benzoyl-6-nitrocoumarin, 3-benzoyl-7-diethylaminocoumarin, 7-dimethylamino-3-(4-methoxybenzoyl)coumarin, 7- diethylamino-3-(4-methoxybenzoyl)coum arin, 7- diethylamino - 3 - (4- diethylamino)coumarin, 7-methoxy-3-(4-methoxybenzoyl)coumarin, 3-(4-nitrobenzoyl)benzo[f]coumarin, 3-(4-ethoxycinnamoyl)-7-methoxycoumarin, 3-(4-dimethylaminocinnamoyl)coumarin, 3-(4-diphenylaminocinnamoyl)coumarin, 3-[(3-dimethylbenzothiazol-2-ylidene)acetyl]coumarin, 3-[(1-methylnaphtho[1,2-d]thiazol-2-ylidene)acetyl]coumarin, 3,3'-carbonyl bis(6-methoxycoumarin), 3,3'-carbonyl bis(7-acetoxycoumarin), 3,3'-carbonyl bis(7-dimethylaminocoumarin), 3-(2-benzothiazoyl)-7-(diethylamino)coumarin, 3-(2-benzothiazoyl)-7-(dibutylamino)coumarin, 3-(2-benzoimidazoyl)-7-(diethylamino)coumarin, 3-(2-benzothiazoyl)-7-(dioctylamino)coumarin, 3-acetyl-7-(dimethylamino)coumarin, 3,3'-carbonyl bis(7-dibutylaminocoumarin), 3,3'-carbonyl-7-diethylaminocoumarin-7'-bis(butoxyethyl)aminocoumarin, 10-[3-[4-(dimethylamino)phenyl]-1-oxo-2-propenyl]-2,3,6,7-tetrahydro-1,1,7,7-tetram ethyl-1H,5H,11H-[1]benzopyrrano[6,7,8-ij]quinolizin-11-one, and 10-(2-benzothiazoyl)-2,3,6,7-tetrahydro-1,1,7,7-tetramethyl-1H,5H,11H-[1]benzopyrr ano[6,7,8-ij]quinolizin-11-one.

Particularly preferred among these coumarin compounds are 3,3'-carbonyl bis(7-diethylaminocoumarin), and 3,3'-carbonyl bis(7-dibutylaminocoumarin).

The (bis)acylphosphine oxides, α-diketones, and coumarin compounds in these photopolymerization initiators are desirable in their ability to initiate polymerization in the visible and near ultraviolet regions, and enables polymerization to start with a light source such as a halogen lamp, a light emitting diode (LED), or a xenon lamp. This makes it easier to obtain a prepolymer (C).

The chemical polymerization initiators are preferably organic peroxides. The organic peroxides are not particularly limited, and known organic peroxides may be used. Typical examples of such organic peroxides include ketone peroxides, hydroperoxides, diacyl peroxides, dialkyl peroxides, peroxyketals, peroxyesters, and peroxydicarbonates.

Examples of the ketone peroxides include methyl ethyl ketone peroxide, methyl isobutyl ketone peroxide, methylcyclohexanone peroxide, and cyclohexanone peroxide.

### Examples of the hydroperoxides include

2,5-dimethylhexane-2,5-dihydroperoxide, diisopropylbenzenehydroperoxide, cumenehydroperoxide, t-butylhydroperoxide, and 1,1,3,3-tetramethylbutylhydroperoxide.

Examples of the diacyl peroxides include acetyl peroxide, isobutyryl peroxide, benzoyl peroxide, decanoyl peroxide, 3,5,5-trimethylhexanoyl peroxide, 2,4-dichlorobenzoyl peroxide, and lauroyl peroxide.

Examples of the dialkyl peroxides include di-t-butyl peroxide, dicumyl peroxide, t-butylcumyl peroxide, 2,5-dimethyl-2,5-di(t-butylperoxy)hexane, 1,3-bis(t-butylperoxyisopropyl)benzene, and 2,5-dimethyl-2,5-di(t-butylperoxy)-3-hexyne.

Examples of the peroxyketals include 1,1-bis(t-butylperoxy)-3,3,5-trimethylcyclohexane, 1,1-bis(t-butylperoxy)cyclohexane, 2,2-bis(t-butylperoxy)butane, 2,2-bis(t-butylperoxy)octane, and n-butyl 4,4-bis(t-butylperoxy)valerate.

Examples of the peroxyesters include α-cumyl peroxyneodecanoate, t-butyl peroxyneodecanoate, t-butyl peroxypivalate, 2,2,4-trimethylpentyl peroxy-2-ethylhexanoate, t-amyl peroxy-2-ethylhexanoate, t-butyl peroxy-2-ethylhexanoate, di-t-butyl peroxyisophthalate, di-t-butyl peroxyhexahydroterephthalate, t-butyl peroxy-3,3,5-trimethylhexanoate, t-butyl peroxyacetate, t-butyl peroxybenzoate, and t-butyl peroxymaleic acid.

Examples of the peroxydicarbonates include di-3-methoxyperoxydicarbonate, di-2-ethylhexylperoxydicarbonate, bis(4-t-butylcyclohexyl)peroxydicarbonate, diisopropylperoxydicarbonate, di-n-propylperoxydicarbonate, di-2-ethoxyethylperoxydicarbonate, and diallylperoxydicarbonate.

From an overall balance of safety, storage stability, and radical generating potential, preferred among these organic peroxides are diacyl peroxides, more preferably benzoyl peroxide.

The chemical polymerization initiators may be, for example, an azonitrile initiator, such as 2,2'-azobis(isobutyronitrile), 2,2'-azobis(2,4-dimethylvaleronitrile), 2,2'-azobis(4-methoxy-2,4-dimethylvaleronitrile), or 2,2'-azobis(2-cyclopropylpropionitrile).

The amount of polymerization initiator (d) used for the production of prepolymer (C) is not particularly limited. However, in view of properties such as curability of the prepolymer (C) produced, the preferred amount is 0.1 to 20 parts by mass, more preferably 0.5 to 10 parts by mass, relative to total 100 parts by mass of the polyfunctional monomer (a) and monofunctional monomer (b).

### Organic Solvent (e)

The organic solvent (e) used for the production of prepolymer (C) is preferably one capable of readily dissolving the polymerizable monomers (e.g., polyfunctional monomer (a), monofunctional monomer (b)), the chain transfer agent (c), and the polymerization initiator (d) without reacting with these components. Examples of the organic solvent (e) include aliphatic hydrocarbons (e.g., hexane, heptane), aromatic hydrocarbons (e.g., toluene, xylene), and cyclic ethers (e.g., tetrahydrofuran, dioxane). The organic solvent (e) may be used alone, or two or more thereof may be used in combination. Considering properties such as solubility and ease of removal, the organic solvent (e) is most preferably toluene.

The organic solvent (e) is used in an amount that is preferably 1 to 20 times, more preferably 2 to 10 times, even more preferably 3 to 8 times the total mass of the polyfunctional monomer (a) and monofunctional monomer (b). Gelation may occur during polymerization when the amount of organic solvent (e) is too small. With an overly large amount of organic solvent (e), production of prepolymer (C) may take a long time.

### Polymerization Accelerator (f)

A polymerization accelerator (f) may additionally be used for the production of prepolymer (C). Examples of the polymerization accelerator (f) include amines, sulfinic acids (including salts), derivatives of barbituric acid, triazine compounds, copper compounds, tin compounds, vanadium compounds, halogen compounds, aldehydes, thiol compounds, sulfites, bisulfites, and thiourea compounds. The polymerization accelerator (f) may be used alone, or two or more thereof may be used in combination.

The amines can be classified into aliphatic amines and aromatic amines. Examples of the aliphatic amines include primary aliphatic amines such as n-butylamine, n-hexylamine, and n-octylamine; secondary aliphatic amines such as diisopropylamine, dibutylamine, and N-methylethanolamine; and tertiary aliphatic amines such as N-methyldiethanolamine, N-ethyldiethanolamine, N-n-butyldiethanolamine, N-lauryldiethanolamine, 2 -(dimethylamino)ethyl methacrylate, N-methyldiethanolamine dimethacrylate, N-ethyldiethanolamine dimethacrylate, triethanolamine monomethacrylate, triethanolamine dimethacrylate, triethanolamine trimethacrylate, triethanolamine, trimethylamine, triethylamine, and tributylamine. In view of curability and storage stability of the dental composition, preferred are tertiary aliphatic amines, more preferably N-methyldiethanolamine and triethanolamine.

Examples of the aromatic amines include N,N-bis(2-hydroxyethyl)-3,5-dimethylaniline, N,N-bis(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-diisopropylaniline, N,N-bis(2-hydroxyethyl)-3,5-di-t-butylaniline, N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, ethyl 4-(N,N-dimethylamino)benzoate, methyl 4-(N,N-dimethylamino)benzoate, 2-butoxyethyl 4-(N,N-dimethylamino)benzoate, 2-((meth)acryloyloxy)ethyl 4-(N,N-dimethylamino)benzoate, 4-(N,N-dimethylamino)benzophenone, and butyl 4-(N,N-dimethylamino)benzoate. In view of the ability to impart desirable curability to the dental composition, preferred are N,N-bis(2-hydroxyethyl)-p-toluidine, ethyl 4-(N,N-dimethylamino)benzoate, 2-butoxyethyl 4-(N,N-dimethylamino)benzoate, and 4-(N,N- dimethylamino)benzophenone.

Examples of the sulfinic acids include p-toluenesulfinic acid, sodium p-toluenesulfinate, potassium p-toluenesulfinate, lithium p-toluenesulfinate, calcium p-toluenesulfinate, benzenesulfinic acid, sodium benzenesulfinate, potassium benzenesulfinate, lithium benzenesulfinate, calcium benzenesulfinate, 2,4,6-trimethylbenzenesulfinic acid, sodium 2,4,6-trimethylbenzenesulfinate, potassium 2,4,6-trimethylbenzenesulfinate, lithium 2,4,6-trimethylbenzenesulfinate, calcium 2,4,6-trimethylbenzenesulfinate, 2,4,6-triethylbenzenesulfinic acid, sodium 2,4,6-triethylbenzenesulfinate, potassium 2,4,6-triethylbenzenesulfinate, lithium 2,4,6-triethylbenzenesulfinate, calcium 2,4,6 -triethylbenzenesulfinate, 2,4,6-triisopropylbenzenesulfinic acid, sodium 2,4,6-triisopropylbenzenesulfinate, potassium 2,4,6-triisopropylbenzenesulfinate, lithium 2,4,6-triisopropylbenzenesulfinate, and calcium 2,4,6-triisopropylbenzenesulfinate. Particularly preferred are sodium benzenesulfinate, sodium p-toluenesulfinate, and sodium 2,4,6-triisopropylbenzenesulfinate.

Examples of the derivatives of barbituric acid include barbituric acid, 1,3-dimethylbarbituric acid, 1,3-diphenylbarbituric acid, 1,5-dimethylbarbituric acid, 5-butylbarbituric acid, 5-ethylbarbituric acid, 5-isopropylbarbituric acid, 5-cyclohexylbarbituric acid, 1,3,5-trimethylbarbituric acid, 1,3-dimethyl-5-ethylbarbituric acid, 1,3-dimethyl-5-n-butylbarbituric acid, 1,3-dimethyl-5-isobutylbarbituric acid, 1,3-dimethyl-5-cyclopentylbarbituric acid, 1,3-dimethyl-5-cyclohexylbarbituric acid, 1,3-dimethyl-5-phenylbarbituric acid, 1-cyclohexyl-1-ethylbarbituric acid, 1-benzyl-5-phenylbarbituric acid, 5-methylbarbituric acid, 5-propylbarbituric acid, 1,5-diethylbarbituric acid, 1-ethyl-5-methylbarbituric acid, 1-ethyl-5-isobutylbarbituric acid, 1,3-diethyl-5-butylbarbituric acid, 1-cyclohexyl-5-methylbarbituric acid, 1-cyclohexyl-5-ethylbarbituric acid, 1-cyclohexyl-5-octylbarbituric acid, 1-cyclohexyl-5-hexylbarbituric acid, 5-butyl-1-cyclohexylbarbituric acid, 1-benzyl-5-phenylbarbituric acid, thiobarbituric acid, and salts thereof. Examples of the salts of the derivatives of barbituric acid include alkali metal salts, and alkali-earth metal salts, specifically, sodium 5-butylbarbiturate, sodium 1,3,5-trimethylbarbiturate, and sodium 1-cyclohexyl-5-ethylbarbiturate.

Particularly preferred as derivatives of barbituric acid are 5-butylbarbituric acid, 1,3,5-trimethylbarbituric acid, 1-cyclohexyl-5-ethylbarbituric acid, 1-benzyl-5-phenylbarbituric acid, and sodium salts of these.

Examples of the triazine compounds include 2,4,6-tris(trichloromethyl)-s-triazine, 2,4,6-tris(tribromomethyl)-s-triazine, 2-methyl-4,6-bis(trichloromethyl)-s-triazine, 2-methyl-4,6-bis(tribromomethyl)-s-triazine, 2-phenyl-4,6-bis(trichloromethyl)-s -triazine, 2-(p-methoxyphenyl)-4,6-bis(trichloromethyl)-s-triazine, 2-(p-methylthiophenyl)-4,6-bis(trichloromethyl)-s-triazine, 2-(p-chlorophenyl)-4,6-bis(trichloromethyl)-s-triazine, 2-(2,4-dichlorophenyl)-4,6-bis(trichloromethyl)-s-triazine, 2-(p-bromophenyl)-4,6-bis(trichloromethyl)-s-triazine, 2-(p-tolyl)-4,6-bis(trichloromethyl)-s-triazine, 2-n-propyl-4,6-bis(trichloromethyl)-s-triazine, 2-(α,α,β-trichloroethyl)-4,6-bis(trichloromethyl)-s-triazine, 2-styryl-4,6-bis(trichloromethyl)-s -triazine, 2-[2-(p-methoxyphenyl)ethenyl]-4,6-bis(trichloromethyl)-s-triazine, 2-[2-(o-methoxyphenyl)ethenyl]-4,6-bis(trichloromethyl)-s-triazine, 2-[2-(p-butoxyphenyl)ethenyl]-4,6-bis(trichloromethyl)-s-triazine, 2-[2-(3,4-dimethoxyphenyl)ethenyl]-4,6-bis(trichloromethyl)-s-triazine, 2-[2-(3,4,5-trimethoxyphenyl)ethenyl]-4,6-bis(trichloromethyl)-s-triazine, 2-(1-naphthyl)-4,6-bis(trichloromethyl)-s-triazine, 2-(4-biphenylyl)-4,6-bis(trichloromethyl)-s-triazine, 2-[2-{N,N-bis(2-hydroxyethyl)amino}ethoxy]-4,6-bis(trichloromethyl)-s-triazine, 2-[2-{N-hydroxyethyl-N-ethylamino}ethoxy]-4,6-bis(trichloromethyl)-s-triazine, 2-[2-{N-hydroxyethyl-N-methylamino}ethoxy]-4,6-bis(trichloromethyl)-s-triazine, and 2-[2-{N,N-diallylamino}ethoxy]-4,6-bis(trichloromethyl)-s-triazine.

Among these triazine compounds, preferred for polymerization activity is 2,4,6-tris(trichloromethyl)-s-triazine. Preferred for storage stability are 2-phenyl-4,6-bis(trichloromethyl)-s -triazine, 2-(p-chlorophenyl)-4,6-bis(trichloromethyl)-s-triazine, and 2-(4-biphenylyl)-4,6-bis(trichloromethyl)-s-triazine. The triazine compounds may be used alone, or two or more thereof may be used in combination.

Examples of the copper compounds include copper acetylacetonate, copper(II) acetate, copper oleate, copper(II) chloride, and copper(II) bromide.

Examples of the tin compounds include di-n-butyltin dimaleate, di-n-octyltin dimaleate, di-n-octyltin dilaurate, and di-n-butyltin dilaurate. Preferred are di-n-octyltin dilaurate, and di-n-butyltin dilaurate.

The vanadium compounds are preferably vanadium compounds with valences of IV and V Examples of vanadium compounds with valences of IV and V include vanadium(IV) oxide, vanadium(IV) oxy acetylacetonate, vanadyl oxalate, vanadyl sulfate, vanadium(IV) oxobis(1-phenyl-1,3-butanedionate), bis(maltolato)oxovanadium(IV), vanadium(V) oxide, sodium metavanadate, and ammonium metavanadate.

Examples of the halogen compounds include dilauryldimethylammonium chloride, lauryldimethylbenzylammonium chloride, benzyltrimethylammonium chloride, tetramethylammonium chloride, benzyldimethylcetylammonium chloride, and dilauryldimethylammonium bromide.

Examples of the aldehydes include terephthalaldehyde, and derivatives of benzaldehyde. Examples of the derivatives of benzaldehyde include dimethylaminobenzaldehyde, p-methyloxybenzaldehyde, p-ethyloxybenzaldehyde, and p-n-octyloxybenzaldehyde. In view of curability, p-n-octyloxybenzaldehyde is preferred.

Examples of the thiol compounds include 3-mercaptopropyltrimethoxysilane, 2-mercaptobenzooxazole, decanethiol, and thiobenzoic acid.

Examples of the sulfites include sodium sulfite, potassium sulfite, calcium sulfite, and ammonium sulfite.

Examples of the bisulfites include sodium bisulfite and potassium bisulfite.

Examples of the thiourea compounds include 1-(2-pyridyl)-2-thiourea, thiourea, methylthiourea, ethylthiourea, N,N'-dimethylthiourea, N,N'-diethylthiourea, N,N'-di-n-propylthiourea, N,N'-dicyclohexylthiourea, trimethylthiourea, triethylthiourea, tri-n-propylthiourea, tricyclohexylthiourea, tetramethylthiourea, tetraethylthiourea, tetra-n-propylthiourea, and tetracyclohexylthiourea.

The amount of polymerization accelerator (f) used for the production of prepolymer (C) is not particularly limited. However, in view of properties such as the curability of the prepolymer (C) produced, the polymerization accelerator (f) is used in an amount of preferably 0.1 to 20 parts by mass, more preferably 0.5 to 10 parts by mass relative to total 100 parts by mass of the polyfunctional monomer (a) and monofunctional monomer (b).

### Polymerization Inhibitor (g)

A polymerization inhibitor (g) may additionally be used for the production of prepolymer (C). As an example, the polymerization inhibitor (g) may be used to improve the storage stability of the prepolymer (C) produced, or to regulate or stop the polymerization reaction. The polymerization inhibitor may be added to the reaction system before starting polymerization, or may be added during or after polymerization. Examples of the polymerization inhibitor (g) include 3,5-di-t-butyl-4-hydroxytoluene, hydroquinone, dibutylhydroquinone, dibutylhydroquinonemonomethyl ether, hydroquinonemonomethyl ether, and 2,6-di-t-butylphenol. The polymerization inhibitor (g) may be used alone, or two or more thereof may be used in combination.

### Other Additives

Aside from the polymerizable monomers (e.g., polyfunctional monomer (a), monofunctional monomer (b)), the chain transfer agent (c), the polymerization initiator (d), the organic solvent (e), the polymerization accelerator (f), and the polymerization inhibitor (g) used for the production of prepolymer (C), it is also possible to optionally add other additives, for example, such as ultraviolet absorbers, antioxidants, antimicrobial agents, dispersants, pH adjusters, fluorescent agents, pigments, and dyes.

The prepolymer (C) obtained after polymerization in the manner described above can be collected using a known method, for example, such as vacuum drying, devolatilization under heating, freeze drying, or reprecipitation. After collection, the prepolymer (C) may be optionally purified using a known method such as washing or reprecipitation.

### Polymerization Initiator (D)

The polymerization initiator (d) that can be used for the production of prepolymer (C) can preferably be used as the polymerization initiator (D) contained in a dental composition of the present invention. Accordingly, any overlapping features will not be described. The polymerization initiator (D) may be used alone, or two or more thereof may be used in combination.

The content of polymerization initiator (D) in a dental composition of the present invention is not particularly limited. However, in view of curability and other properties of the dental composition obtained, the content of polymerization initiator (D) is preferably 0.001 mass% or more, more preferably 0.01 mass% or more, even more preferably 0.02 mass% or more, particularly preferably 0.1 mass% or more relative to the mass of the dental composition. Because an overly high content of polymerization initiator (D) may cause the polymerization initiator (D) to precipitate from the dental composition, the content of polymerization initiator (D) is preferably 30 mass% or less, more preferably 20 mass% or less, even more preferably 15 mass% or less, particularly preferably 10 mass% or less, and may be 5 mass% or less, 2 mass% or less, or 1 mass% or less, relative to the mass of the dental composition.

### Other Filler (E)

A dental composition of the present invention may further comprise a filler (E) other than the inorganic filler (B). The filler (E) may be any of various types of fillers that do not classify as the inorganic filler (B). Typically, such fillers can be broadly classified into inorganic filler (E-1), organic filler (E-2) (excluding those exemplified for prepolymer (C)), and organic-inorganic composite filler (E-3) (a filler containing an inorganic filler and a polymer of a polymerizable monomer). The filler (E) may be used alone, or two or more thereof may be used in combination. When used in combination, the filler (E) may be used with another filler (E) differing in, for example, material, particle size distribution, and form. The filler (E) may be a commercially available product. The filler (E) is preferably an inorganic filler (E-1).

Examples of the inorganic filler (E-1) include various types of glasses. (For example, glasses containing silica as a main component, and, optionally, oxides of elements such as heavy metals, boron, and aluminum. Examples include glass powders of common compositions, for example, such as fused silica, quartz, soda-lime-silica glass, E glass, C glass, and borosilicate glass (PYREX® glass); and dental glass powders, such as barium glass (for example, GM27884 and 8235 manufactured by Schott, and E-2000 and E-3000 manufactured by Esstech), strontium-borosilicate glass (for example, E-4000 manufactured by Esstech), lanthanum glass-ceramics (for example, GM31684 manufactured by Schott), and fluoroaluminosilicate glass (for example, GM35429, G018-091, and G018-117 manufactured by Schott)). Other examples of inorganic filler (E-1) include alumina, ceramics, silica-based composite oxides (e.g., silica-titania, silica-zirconia), diatomaceous earth, kaolin, clay minerals (e.g., montmorillonite), activated earth, synthetic zeolite, mica, calcium fluoride, ytterbium fluoride, yttrium fluoride, calcium phosphate, barium sulfate, zirconium dioxide (zirconia), titanium dioxide (titania), and hydroxyapatite. The inorganic filler (E-1) may be used alone, or two or more thereof may be used in combination. The inorganic filler (E-1) is preferably one containing silica as a main component (containing at least 5 mass%, preferably at least 10 mass% silica).

The shape of inorganic filler (E-1) is not particularly limited, and the inorganic filler (E-1) may be used in the form of a powder of irregularly shaped or spherical particles. An irregularly shaped inorganic filler (E-1) improves the mechanical strength and wear resistance of the cured product obtained. A spherical inorganic filler (E-1) improves the gloss polishability and gloss retention of the cured product obtained. The shape of inorganic filler (E-1) may be appropriately selected according to the intended use of the dental composition.

The inorganic filler (E-1) has an average particle diameter of preferably 0.001 to 50 µm, more preferably 0.01 to 10 µm, even more preferably 0.1 to 5 µm, particularly preferably 0.15 to 3 µm.

The inorganic filler (E-1) may be an agglomerated particle formed by particle agglomeration. Commercially available inorganic fillers typically exist in the form of aggregates. The cohesion of commercially available inorganic fillers is so weak that these fillers break into particle sizes indicated by the manufacturer when 10 mg of its powder is added and ultrasonically dispersed at 40 W and 39 KHz for 30 minutes in 300 mL of water or in the same amount of a dispersion medium prepared by adding a surfactant (e.g., at most 5 mass% of sodium hexametaphosphate) to water. In contrast, the particles in the agglomerate mentioned above are strongly held together, and become hardly dispersed even under these conditions.

Preferably, the inorganic filler (E-1) is subjected to a surface treatment in advance with a surface treatment agent, in order to improve the mechanical strength of cured product by improving compatibility with the polymerizable monomer (A) or chemical bonding with the polymerizable monomer (A). The surface treatment agent may be a known surface treatment agent, or a surface treatment agent that can be used for the surface treatment of inorganic filler (B), such as those mentioned above. Specific examples of the preferred surface treatment agents include vinyltrimethoxysilane, vinyltriethoxysilane, vinyltrichlorosilane, vinyltri(β-methoxyethoxy)silane, 3-methacryloyloxypropyltrimethoxysilane, 11-methacryloyloxyundecyltrimethoxysilane, 3-glycidoxypropyltrimethoxysilane, 3-mercaptopropyltrimethoxysilane, and 3-aminopropyltriethoxysilane. The amount of surface treatment agent is not particularly limited. However, the surface treatment agent is used in an amount of preferably 0.1 to 30 parts by mass, more preferably 1 to 20 parts by mass relative to 100 parts by mass of the inorganic filler (E-1) before surface treatment.

Examples of materials of the organic filler (E-2) include polymethylmethacrylate, polyethylmethacrylate, a methyl methacrylate-ethyl methacrylate copolymer, crosslinked polymethylmethacrylate, crosslinked polyethylmethacrylate, polyamides, polyvinyl chloride, polystyrene, chloroprene rubber, nitrile rubber, an ethylene-vinyl acetate copolymer, a styrene-butadiene copolymer, an acrylonitrile-styrene copolymer, and an acrylonitrile-styrene-butadiene copolymer. These may be used alone, or two or more thereof may be used in combination. In view of considerations such as ease of handling of the dental composition and mechanical strength, the average particle diameter of the organic filler (E-2) is preferably 0.0005 to 50 µm, more preferably 0.001 to 10 µm.

The organic-inorganic composite filler (E-3) is preferably one in which inorganic particles having an average particle diameter of 0.5 µm or less are dispersed in an organic matrix. The method of preparation is not particularly limited. For example, the organic-inorganic composite filler (E-3) can be prepared by adding a known polymerizable monomer and a known polymerization initiator to the inorganic filler (E-1), polymerizing the filler mixture in paste form by a polymerization method such as solution polymerization, suspension polymerization, emulsion polymerization, or bulk polymerization, and pulverizing the resulting polymer.

The organic-inorganic composite filler (E-3) has an average particle diameter of preferably 1 to 50 µm, more preferably 3 to 25 µm. By setting these lower limits for the average particle diameter of organic-inorganic composite filler (E-3), the dental composition obtained becomes less sticky, and ease of handling improves. By setting the foregoing upper limits for the average particle diameter of organic-inorganic composite filler (E-3), the dental composition obtained can have reduced roughness and reduced dryness, and ease of handling improves.

When a dental composition of the present invention is containing the filler (E), the content of filler (E) is not particularly limited. However, in view of ease of handling of the dental composition and the mechanical strength of the cured product, the content of filler (E) is preferably 1 mass% or more, more preferably 5 mass% or more, even more preferably 10 mass% or more, particularly preferably 20 mass% or more, and is preferably 80 mass% or less, more preferably 60 mass% or less, even more preferably 40 mass% or less, relative to the mass of the dental composition.

### Polymerization Accelerator (F)

A dental composition of the present invention may further comprise a polymerization accelerator (F). The polymerization accelerator (f) that can be used for the production of prepolymer (C) can preferably be used as the polymerization accelerator (F). Accordingly, any overlapping features will not be described. The polymerization accelerator (F) may be used alone, or two or more thereof may be used in combination.

When a dental composition of the present invention is containing the polymerization accelerator (F), the content of polymerization accelerator (F) is not particularly limited. However, in view of properties such as the curability of the dental composition obtained, the content of polymerization accelerator (F) is preferably 0.001 mass% or more, more preferably 0.01 mass% or more, even more preferably 0.02 mass% or more, and may be 0.03 mass% or more, 0.05 mass% or more, or 0.1 mass% or more, relative to the mass of the dental composition. Because an overly high content of polymerization accelerator (F) may cause the polymerization accelerator (F) to precipitate from the dental composition, the content of polymerization accelerator (F) is preferably 30 mass% or less, more preferably 20 mass% or less, even more preferably 10 mass% or less, particularly preferably 5 mass% or less, and may be 2 mass% or less, 1 mass% or less, or 0.5 mass% or less, relative to the mass of the dental composition.

### Polymerization Inhibitor (G)

A dental composition of the present invention may further comprise a polymerization inhibitor (G). The polymerization inhibitor (g) that can be used for the production of prepolymer (C) can preferably be used as the polymerization inhibitor (G). Accordingly, any overlapping features will not be described. The polymerization inhibitor (G) may be used alone, or two or more thereof may be used in combination.

When a dental composition of the present invention is containing the polymerization inhibitor (G), the content of polymerization inhibitor (G) is not particularly limited. However, the content of polymerization inhibitor (G) is preferably 0.0001 mass% or more, more preferably 0.001 mass% or more, even more preferably 0.01 mass% or more, and is preferably 1 mass% or less, more preferably 0.1 mass% or less, even more preferably 0.03 mass% or less, relative to the mass of the dental composition.

### Other Components

Aside from the polymerizable monomer (A), inorganic filler (B), prepolymer (C), polymerization initiator (D), filler (E), polymerization accelerator (F), and polymerization inhibitor (G), a dental composition of the present invention may optionally comprise other components such as solvents, ultraviolet absorbers, antioxidants, antimicrobial agents, dispersants, pH adjusters, fluorescent agents, pigments, and dyes.

For advantages such as enhancement of the effectiveness of the present invention, the total content of the polymerizable monomer (A), inorganic filler (B), prepolymer (C), polymerization initiator (D), filler (E), polymerization accelerator (F), and polymerization inhibitor (G) in a dental composition of the present invention is preferably 50 mass% or more, more preferably 80 mass% or more, even more preferably 95 mass% or more, particularly preferably 98 mass% or more, and may be 100 mass%.

### Method of Production of Dental Composition

A method of preparation of a dental composition of the present invention is not particularly limited, and a dental composition of the present invention can be obtained by combining the components in predetermined amounts. The components may be combined in any order, at once or in two or more separate portions. Optionally, the components may be mixed or kneaded, or may be subjected to degassing, for example, vacuum degassing. The resultant dental composition may be charged into a single container (e.g., a syringe) to prepare a one-pack type dental composition.

### Uses

A dental composition of the present invention is not limited to particular uses, and may be used as a variety of dental materials. Specifically, a dental composition of the present invention can be suitably used as, for example, a dental composite resin (for example, a composite resin for filling cavities, a composite resin for abutment construction, a composite resin for dental caps), a denture base resin, a denture base liner, an impression material, a luting material (for example, a resin cement, a resin-added glass ionomer cement), a dental bonding agent (for example, an orthodontics adhesive, an adhesive for application to cavities), a tooth fissure sealant, a resin block for CAD/CAM, a temporary crown, or an artificial teeth material. Because of desirable properties such as good ease of handling, highly stable consistency, low polymerization shrinkage stress, and improved mechanical strength and polishability for the cured product, a dental composition of the present invention is particularly suited as a dental composite resin.

The present invention encompasses embodiments combining the foregoing features, provided that such combinations made in various forms within the technical idea of the present invention can produce the effects of the present invention.

### EXAMPLES

The following describes the present invention in greater detail by way of Examples and Comparative Examples. It is to be noted, however, that the present invention is not limited to the following Examples. The following summarizes details of Examples, including the test methods and materials used in Examples.

### Test Methods

### Average Particle Diameter of Inorganic Filler

Inorganic fillers obtained in the Production Examples below were ashed at 450°C for 4 hours using an electric furnace, and each was measured for average particle diameter with a laser diffraction particle size distribution analyzer (SALD-2100, Shimadzu Corporation), using ethanol as a dispersion medium.

### Specific Surface Area of Inorganic Filler

Inorganic fillers obtained in the Production Examples below were ashed at 450°C for 4 hours using an electric furnace. After vacuum devolatilization at 100°C for 2 hours, the inorganic fillers were each was measured for specific surface area with a specific surface area measurement device (BELSORP-mini II manufactured by MicrotracBEL Corp.) according to the BET method performed at a measurement temperature of 77 K using nitrogen as adsorbate gas. For the measurement, a multipoint BET analysis was conducted with five points on an adsorption isotherm in the P/Po range of 0.05 to 0.3, where P is the adsorbate equilibrium pressure (kPa), and Po is the saturated vapor pressure (kPa).

### Hydroxyl Number of Prepolymer

The hydroxyl number of prepolymer was measured in compliance with the method described in JIS K 1557-1:2007.

### Weight-Average Molecular Weight of Prepolymer

The weight-average molecular weight of prepolymer was determined by GPC analysis. Specifically, tetrahydrofuran was used as eluent, and a column was prepared by joining two TSKgel SuperMultipore HZM-M columns (Tosoh Corporation) and one TSKgel SuperHZ 4000 column (Tosoh Corporation), end to end. A GPC system HLC-8320 (manufactured by Tosoh Corporation) equipped with a differential refractive index detector (RI detector) was used as GPC device. For measurement, 4 mg of a prepolymer was dissolved in 5 mL of tetrahydrofuran to prepare a sample solution. With the column oven temperature set to 40°C, 20 µL of sample solution was injected at an eluent flow rate of 0.35 mL/min, and the resulting chromatogram of the prepolymer was analyzed. Separately, a standard curve relating retention time to molecular weight was created by GPC using ten polystyrene standards having a molecular weight in the 400 to 5,000,000 range. The weight-average molecular weight of prepolymer was then determined from its chromatogram, using the standard curve.

### Number of Unreacted Polymerizable Functional Groups in Prepolymer (Average Number per Molecule)

The concentration p (mol/g) of unreacted polymerizable functional groups in a prepolymer was determined by ¹H-NMR analysis, and multiplied by the measured weight-average molecular weight (M_{w}) (ρ × M_{w}) to find the number of unreacted polymerizable functional groups in the prepolymer (average number per molecule).

For the ¹H-NMR analysis, about 30 mg of weighed prepolymer and about 2 mg of weighed dimethyl terephthalate (internal standard; a molecular weight of 194.19) were dissolved in 3 mL of deuterated chloroform (W_{P}: weight of prepolymer in mg; W_{D}: weight of dimethyl terephthalate in mg). The sample was measured at room temperature by being scanned 16 times with a nuclear magnetic resonance apparatus (ULTRA SHIELD 400 PLUS manufactured by Bruker), and the mole ratio (R_{P/D}) of methacryloyl group and dimethyl terephthalate was determined from the integral values of peaks (5.55 ppm and 6.12 ppm) attributed to protons derived from the methacryloyl group, and the integral value of a peak (8.10 ppm) attributed to protons in the aromatic group of dimethyl terephthalate (R_{P/D} = [(I_{5.55} + I_{6.12})/2]/(I_{8.10}/4), where I_{5.55} represents the integral value of a peak at 5.55 ppm, I_{6.12} the integral value of a peak at 6.12 ppm, and I_{8.10} the integral value of a peak at 8.10 ppm). The calculated value of R_{P/D} was then used to determine the concentration p [mol/g] of polymerizable functional groups in the prepolymer (p = [R_{P/D} × W_{D}/194.19]/W_{P}).

### Flexural Strength of Cured Product

The dental compositions obtained in Examples and Comparative Examples were degassed in vacuum, and each was charged into a stainless-steel mold (dimensions: 2 mm × 2 mm × 25 mm). With the dental composition being pressed between glass slides from top and bottom, light was applied through the glass slides from both sides to cure the dental composition and obtain a cured product specimen. Here, light was applied at 5 points each side, 10 seconds at each point, using a dental LED photoirradiator for polymerization (PenCure 2000 manufactured by J. Morita Corp.). A total of five cured products were prepared for each Example and Comparative Example. The cured product was stored in 37°C distilled water for 24 hours after being taken out of the mold. For the measurement of flexural strength, the specimens were tested in a three-point flexural test conducted in compliance with JIS T 6514:2015 and ISO 4049:2009 at a span length of 20 mm and a crosshead speed of 1 mm/min, using a precision universal testing machine (Autograph AG-I, 100 kN, manufactured by Shimadzu Corporation). From the measured values, a mean value was calculated for each specimen to find the flexural strength. The flexural strength is preferably 100 MPa or more, more preferably 110 MPa or more, even more preferably 120 MPa or more, particularly preferably 130 MPa or more. The upper limits of flexural strength are not particularly limited, and may be, for example, 200 MPa or less, or 150 MPa or less.

### Polishability of Cured Product

The dental composition obtained in each Example and Comparative Example was charged into a polytetrafluoroethylene mold (10 mm in inner diameter × 2.0 mm in thickness), and light was applied for 10 seconds using a dental LED photoirradiator for polymerization (PenCure 2000 manufactured by J. Morita Corp.). After photoirradiation, the cured product was taken out of the mold, and a clean smooth surface was polished with #600 abrasive paper under dry conditions. The surface was then polished first with a silicone point, brown (M2 HP, manufactured by Shofu Inc.) at a rotational speed of about 5,000 rpm for 10 seconds, and then with a silicone point, blue (M3 HP, manufactured by Shofu Inc.) at a rotational speed of about 5,000 rpm for 10 seconds, using a laboratory micromotor Volvere RX (manufactured by NSK). The gloss of the polished surface was measured with a gloss meter (VG-2000 manufactured by Nippon Denshoku Industries Co., Ltd.; measurement angle: 60°), and the percentage (percentage gloss) relative to 100% gloss of a mirror was determined as an index of polishability of cured product (n = 3). Tables 2 to 4 show the mean values. The gloss is preferably 30% or more, more preferably 40% or more, even more preferably 50% or more, particularly preferably 60% or more, most preferably 65% or more.

### Ease of Handling

The dental compositions obtained in Examples and Comparative Examples were evaluated for ease of handling as a measure of how easily the dental composition can be handled for filling, using the following criteria (n = 1). Specifically, the dental composition was determined as "Good" when it was only slightly sticky or dry, and easy to handle, and "Excellent" when the dental composition, aside from meeting this criterion, was particularly easy to handle. The dental composition was determined as "Poor" when filling was practically impossible because of heavy stickiness and dryness.

### Stability of Consistency

The dental compositions obtained in Examples and Comparative Examples were divided into two groups. One was stored at 25°C in the dark for 24 hours, whereas the other was stored at 60°C in the dark for 1 week. After storage, 0.5 mL of a dental composition from each group was crushed under a 1 kg load applied for 30 seconds from the top via a glass plate. The dental composition, flat and circular, was then measured for its maximum diameter and minimum diameter, and a mean value of these diameters (mm) was calculated as consistency (n = 1). Larger consistency values mean that the dental composition is softer. The ratio (x/y) of consistency (x) after 1-week storage at 60°C to consistency (y) after 24-hour storage at 25°C was then calculated as the stability of consistency in percent. The preferred range of consistency stability is 80 to 120%, more preferably 85 to 115%, even more preferably 88 to 112%, particularly preferably 90 to 110%, most preferably 92 to 108%.

### Polymerization Shrinkage Stress

The dental composition obtained in each Example and Comparative Example was charged into a ring-shaped mold (stainless steel; 5.5 mm in inner diameter × 0.8 mm in thickness) placed on a 4.0 mm-thick glass plate. The glass plate was used after being sandblasted with an alumina powder having a particle diameter of 50 µm. A stainless-steel jig (∅ = 5 mm), coupled to a universal testing machine (Autograph AG-I, 100 kN, manufactured by Shimadzu Corporation), was then placed on the dental composition filling the mold. The dental composition was cured by applying light for 20 seconds through the glass plate, using a dental LED photoirradiator for polymerization (PenCure 2000 manufactured by J. Morita Corp.). The polymerization shrinkage stress due to curing of the dental composition by a polymerization reaction initiated by photoirradiation was measured with the universal testing machine (n = 3). Tables 2 to 4 show the mean values. Smaller values of polymerization shrinkage stress are preferred because it means that a contraction gap is less likely to occur. Smaller values of polymerization shrinkage stress are also preferred from a procedure standpoint because a larger amount of dental composition can be filled at once when the polymerization shrinkage stress is smaller. The polymerization shrinkage stress is preferably 15.0 MPa or less, more preferably 13.0 MPa or less, even more preferably 12.0 MPa or less, particularly preferably 11.0 MPa or less, most preferably 10.0 MPa or less.

### Materials

### Polymerizable monomer

D2.6E: 2,2-Bis(4-methacryloyloxypolyethoxyphenyl)propane (average number of moles of ethyleneoxy groups added: 2.6)
A-BPEF: 9,9-Bis[4-(2-acryloyloxyethoxy)phenyl]fluorene
UDMA:
   2,2,4-Trimethylhexamethylenebis(2-carbamoyloxyethyl)dimethacrylate
Bis-GMA: 2,2-Bis[4-(3-methacryloyloxy-2-hydroxypropoxy)phenyl]propane 3G: Triethylene glycol dimethacrylate

IBMA: Isobornyl methacrylate
DDMA: Dodecyl methacrylate
PHE-1G: Phenoxyethylene glycol methacrylate

### Chain transfer agent

OT: 1-Octanethiol
3M2B: 3-Mercapto-2-butanol
DPMP: 2,4-Diphenyl-4-methyl-1-pentene

### Polymerization initiator

BAPO: Bis(2,4,6-trimethylbenzoyl)phenylphosphine oxide
BPO: Benzoyl peroxide
CQ: Camphorquinone
TMDPO: 2,4,6-Trimethylbenzoyldiphenylphosphine oxide

### Polymerization accelerator

PDE: Ethyl 4-(N,N-dimethylamino)benzoate

### Polymerization inhibitor

BHT: 3,5-di-t-Butyl-4-hydroxytoluene

### Ultraviolet absorber

TN326: Tinuvin 326 (manufactured by Ciba Specialty Chemicals)

### Inorganic filler

Inorganic fillers produced in the Production Examples below were used.

### Production Example 1

### Production of SiO₂-ZrO₂-1

A solution of 32.5 g of zirconium oxynitrate in 325 g of distilled water was prepared. While stirring the solution, 425 g of a commercially available silica sol (SNOWTEX OL, manufactured by Nissan Chemical Corporation) was gradually added to obtain a mixture. A mixed powder obtained after freeze drying the mixture was placed in an alumina crucible, and was fired at 600°C for 1 hour after being heated at a rate of temperature increase of 2°C/min in an electric furnace. The resulting solid was pulverized for 240 minutes using a planetary ball mill (Classic Line P-6, Zirconia Ball, manufactured by Fritsch GmbH). For hydrophobization, the pulverized powder was surface treated with 3-methacryloyloxypropyltrimethoxysilane used in 10 parts by mass relative to 100 parts by mass of the powder. This produced an inorganic filler (SiO₂-ZrO₂-1).

The inorganic filler (SiO₂-ZrO₂-1) produced had an average particle diameter of 3.0 µm, and a specific surface area of 146 m²/g.

### Production Example 2

### Production of SiO₂-ZrO₂-2

An inorganic filler (SiO₂-ZrO₂-2) was obtained following the method described in Production Example 1 of Patent Literature 1.

The inorganic filler (SiO₂-ZrO₂-2) produced had an average particle diameter of 6.3 µm, and a specific surface area of 182 m²/g.

### Production Example 3

### Production of SiO₂-Yb₂O₃

The water content in a commercially available silica-ytterbium oxide aqueous dispersion (SG-YBSO30SW manufactured by Sukgyung AT) was removed using an evaporator, and the resultant solid component was pulverized with a planetary ball mill (Classic Line P-6, Zirconia Ball, manufactured by Fritsch GmbH) for 180 minutes. The pulverized powder was fired for 1 hour in an electric furnace set at 800°C, and was pulverized for 180 minutes using the planetary ball mill. For hydrophobization, the powder was surface treated with 3-methacryloyloxypropyltrimethoxysilane used in 10 parts by mass relative to 100 parts by mass of the powder. This produced an inorganic filler (SiO₂-Yb₂O₃).

The inorganic filler (SiO₂-Yb₂O₃) produced had an average particle diameter of 5.7 µm, and a specific surface area of 95.8 m²/g.

### Production Example 4

### Production of SiO₂-BaO

A commercially available silica-barium oxide powder (GM27884 NanoFine 180, manufactured by SCHOTT) was fired for 1 hour in an electric furnace set at 700°C, and was pulverized for 60 minutes using a planetary ball mill (Classic Line P-6, Zirconia Ball, manufactured by Fritsch GmbH). For hydrophobization, the powder was surface treated with 3-methacryloyloxypropyltrimethoxysilane used in 10 parts by mass relative to 100 parts by mass of the powder. This produced an inorganic filler (SiO₂-BaO).

The inorganic filler (SiO₂-BaO) produced had an average particle diameter of 4.1 µm, and a specific surface area of 27.8 m²/g.

### Production Example 5

### Production of NF180

A three-neck flask was charged with 100 parts by mass of a commercially available barium glass (GM27884 NanoFine 180, manufactured by Schott), 7 parts by mass of 3-methacryloyloxypropyltrimethoxysilane, and 173 parts by mass of toluene, and the mixture was stirred at room temperature for 2 hours. After removing toluene under reduced pressure, the mixture was vacuum dried at 40°C for 16 hours, and was heated at 90°C for 3 hours to obtain an inorganic filler (NF180) having a surface-treated layer.

The inorganic filler (NF180) produced had an average particle diameter of 0.2 µm, and a specific surface area of 35 m²/g.

### Production Example 6

### Production of UF2.0

A three-neck flask was charged with 100 parts by mass of a commercially available barium glass (UltraFine UF0.2, manufactured by Schott), 5 parts by mass of 3-methacryloyloxypropyltrimethoxysilane, and 173 parts by mass of toluene, and the mixture was stirred at room temperature for 2 hours. After removing toluene under reduced pressure, the mixture was vacuum dried at 40°C for 16 hours, and was heated at 90°C for 3 hours to obtain an inorganic filler (UF2.0) having a surface-treated layer.

The inorganic filler (UF2.0) produced had an average particle diameter of 2.0 µm, and a specific surface area of 3.0 m²/g.

### Prepolymer

Prepolymers produced in the Synthesis Examples below were used.

### Synthesis Examples 1, 2, and 4 to 7

### Synthesis of Prepolymers 1, 2, and 4 to 7

By using polyfunctional monomer (a) and monofunctional monomer (b) as polymerizable monomers in the amounts shown in Table 1, these were charged into a three-neck flask with toluene (used in 5 times the total mass of polyfunctional monomer (a) and monofunctional monomer (b)). After dissolution and 30 minutes of nitrogen bubbling, a chain transfer agent (c) and a polymerization initiator (d) were added in the amounts shown in Table 1, and the mixture was stirred to obtain a toluene solution.

The toluene solution was irradiated with light using a xenon lamp (ProPolymer 3C, Xenon Lamp, manufactured by LUMITECH) to initiate polymerization. After 240 minutes of exposure to light, hexane (used in 6 times the mass of the toluene solution) was dropped, and the precipitate sedimented at the bottom was collected. The precipitate was dried overnight at ordinary temperature under reduced pressure to obtain white powders of prepolymers 1, 2, and 4 to 7.

The prepolymers 1, 2, and 4 to 7 were measured for hydroxyl number according to the method described above. The results are presented in Table 1.

### Synthesis Example 3

### Synthesis of Prepolymer 3

By using polyfunctional monomer (a) and monofunctional monomer (b) as polymerizable monomers in the amounts shown in Table 1, these were charged into a three-neck flask with toluene (used in 5 times the total mass of polyfunctional monomer (a) and monofunctional monomer (b)). After dissolution and 30 minutes of nitrogen bubbling, a chain transfer agent (c) and a polymerization initiator (d) were added in the amounts shown in Table 1, and the mixture was stirred to obtain a toluene solution.

The toluene solution was heat stirred at 80°C under reflux in an oil bath. After 240 minutes of heating, hexane (used in 6 times the mass of the toluene solution) was dropped, and the precipitate sedimented at the bottom was collected. The precipitate was dried overnight at ordinary temperature under reduced pressure to obtain a white powder of prepolymer 3.

The prepolymer 3 was measured for hydroxyl number according to the method described above. The result is presented in Table 1.

**[Table 1]**

| | | Synthesis Example | | | | | | |
|---|---|---|---|---|---|---|---|---|
| | | 1 | 2 | 3 | 4 | 5 | 6 | 7 |
| Polyfunctional monomer (a) | | | | | | | | |
| D2.6E | Mole ratio | 50 | 50 | 50 | | | | 25 |
| A-BPEF | Mole ratio | | | | 50 | | | |
| UDMA | Mole ratio | | | | | 50 | | |
| Bis-GMA | Mole ratio | | | | | | 50 | |
| Monofunctional monomer (b) | | | | | | | | |
| IBMA | Mole ratio | 50 | | | 50 | | 50 | 75 |
| DDMA | Mole ratio | | 50 | | | | | |
| PHE-1G | Mole ratio | | | 50 | | 50 | | |
| Chain transfer agent (c) | | | | | | | | |
| OT | Mole ratio | 60 | | 60 | | 60 | 60 | 60 |
| 3M2B | Mole ratio | | 75 | | | | | |
| DPMP | Mole ratio | | | | 60 | | | |
| Polymerization initiator (d) | | | | | | | | |
| BAPO | Parts by mass^{*1)} | 2.5 | 2.5 | | 2.5 | 2.5 | 2.5 | 2.5 |
| BPO | Parts by mass^{*1)} | | | 2.5 | | | | |
| Prepolymer (C) | | | | | | | | |
| Hydroxyl number | mgKOH/g | 0 | 150 | 0 | 0 | 0 | 180 | 0 |
| weight-average molecular weight | | 44,000 | 25,000 | 40,000 | 57,000 | 48,000 | 65,000 | 13,000 |
| Number of polymerizable functional groups | Number per molecule | 35 | 20 | 38 | 30 | 37 | 25 | 9 |

| | | | | | | | | |
|---|---|---|---|---|---|---|---|---|
| *)Relative to 100 parts by mass of polymerizable monomer | | | | | | | | |

### Other Polymer

LA4285: Acrylic block copolymer Kurarity LA4285, manufactured by Kuraray Co., Ltd.

### Examples 1 to 16 and Comparative Examples 1 to 5

The materials shown in Tables 2 to 4 were used in the proportions shown in Tables 2 to 4. These were mixed in the dark at ordinary temperature (23°C) to prepare dental compositions (pastes). The dental compositions were tested using the methods described above. The results are presented in Tables 2 to 4.

**[Table2]**

| | | Ex. 1 | Com. Ex. 1 | Com. Ex. 2 | Com. Ex. 3 | Ex. 2 | Com. Ex. 4 | Com. Ex. 5 |
|---|---|---|---|---|---|---|---|---|
| Polymerizable monomer composition (X) | Parts by mass | 25 | 25 | 20 | 25 | 25 | 25 | 27 |
| • Polymerizable monomer (A) | | | | | | | | |
| D2.6E | Parts by mass *1) | 70 | 70 | 70 | 70 | 70 | 70 | 70 |
| Bis-GMA | Parts by mass *1) | | | | | | | |
| 3G | Parts by mass *1) | 30 | 30 | 30 | 30 | 30 | 30 | 30 |
| • Polymerization initiator (D) | | | | | | | | |
| CQ | Parts by mass *1) | 0.2 | 0.2 | 0.2 | 0.2 | 0.2 | 0.2 | 0.2 |
| TMDPO | Parts by mass *1) | 0.3 | 0.3 | 0.3 | 0.3 | 0.3 | 0.3 | 0.3 |
| • Polymerization accelerator (F) | | | | | | | | |
| PDE | Parts by mass *1) | 0.2 | 0.2 | 0.2 | 0.2 | 0.2 | 0.2 | 0.2 |
| • Polymerization inhibitor (G) | | | | | | | | |
| BHT | Parts by mass *1) | 0.1 | 0.1 | 0.1 | 0.1 | 0.1 | 0.1 | 0.1 |
| • UV absorber | | | | | | | | |
| TN326 | Parts by mass *1) | 0.1 | 0.1 | 0.1 | 0.1 | 0.1 | 0.1 | 0.1 |
| Prepolymer (C) | | | | | | | | |
| Type | | 1 | - | - | - | 2 | 2 | 2 |
| Content | Parts by mass | 5 | | | | 5 | 5 | 5 |
| Inorganic filler | | | | | | | | |
| SiO₂-ZrO₂-1 | Parts by mass | 70 | 75 | 75 | | 70 | | |
| SiO₂-ZrO₂-2 | Parts by mass | | | | | | | |
| SiO₂-Yb₂O₃ | Parts by mass | | | | | | | |
| SiO₂-BaO | Parts by mass | | | | | | | |
| NF180 | Parts by mass | | | | 5 | | 5 | 68 |
| UF2.0 | Parts by mass | | | | 70 | | 65 | |
| Other polymer | | | | | | | | |
| LA4285 | Parts by mass | | | 5 | | | | |

| Properties of dental composition | | | | | | | | |
|---|---|---|---|---|---|---|---|---|
| Flexural strength of cured product | MPa | 127 | 129 | 95 | 131 | 120 | 136 | 139 |
| Polishability of cured product (Gloss) | % | 64 | 65 | 65 | 24 | 66 | 27 | 67 |
| Ease of handling | | Excellent | Excellent | Good | Excellent | Excellent | Excellent | Poor |
| Consistency stability | % | 92 | 85 | 104 | 89 | 94 | 95 | 96 |
| Polymerization shrinkage stress | MPa | 10.1 | 15.2 | 10.2 | 15.3 | 10.4 | 10.5 | 11.3 |

| | | | | | | | | |
|---|---|---|---|---|---|---|---|---|
| *1)Mass fraction in polymerizable monomer composition (X) | | | | | | | | |

**[Table3]**

| | | Ex. 3 | Ex. 4 | Ex. 5 | Ex. 6 | Ex. 7 | Ex. 8 |
|---|---|---|---|---|---|---|---|
| Polymerizable monomer composition (X) | Parts by mass | 28 | 20 | 29 | 12 | 25 | 25 |
| • Polymerizable monomer (A) | | | | | | | |
| D2.6E | Parts by mass *1) | 70 | 70 | 70 | 70 | 70 | |
| Bis-GMA | Parts by mass *1) | | | | | | 70 |
| 3G | Parts by mass *1) | 30 | 30 | 30 | 30 | 30 | 30 |
| • Polymerization initiator (D) | | | | | | | |
| CQ | Parts by mass *1) | 0.2 | 0.2 | 0.2 | 0.2 | 0.2 | 0.2 |
| TMDPO | Parts by mass *1) | 0.3 | 0.3 | 0.3 | 0.3 | 0.3 | 0.3 |
| • Polymerization accelerator (F) | | | | | | | |
| PDE | Parts by mass *1) | 0.2 | 0.2 | 0.2 | 0.2 | 0.2 | 0.2 |
| • Polymerization inhibitor (G) | | | | | | | |
| BHT | Parts by mass *1) | 0.1 | 0.1 | 0.1 | 0.1 | 0.1 | 0.1 |
| • UV absorber | | | | | | | |
| TN326 | Parts by mass *1) | 0.1 | 0.1 | 0.1 | 0.1 | 0.1 | 0.1 |

| Prepolymer (C) | | | | | | | |
|---|---|---|---|---|---|---|---|
| Type | | 1 | 1 | 1 | 1 | 1 | 1 |
| Content | Parts by mass | 2 | 10 | 8 | 2 | 5 | 5 |

| Inorganic filler | | | | | | | |
|---|---|---|---|---|---|---|---|
| SiO₂-ZrO₂-1 | Parts by mass | 70 | 70 | 63 | 86 | 35 | 70 |
| SiO₂-ZrO₂-2 | Parts by mass | | | | | | |
| SiO₂-Yb₂O₃ | Parts by mass | | | | | | |
| SiO₂-BaO | Parts by mass | | | | | | |
| NF180 | Parts by mass | | | | | 35 | |
| UF2.0 | Parts by mass | | | | | | |

| Other polymer | | | | | | | |
|---|---|---|---|---|---|---|---|
| LA4285 | Parts by mass | | | | | | |

| | | Ex. 3 | Ex. 4 | Ex. 5 | Ex. 6 | Ex. 7 | Ex. 8 |
|---|---|---|---|---|---|---|---|
| Properties of dental composition | | | | | | | |
| Flexural strength of cured product | MPa | 125 | 123 | 112 | 146 | 123 | 136 |
| Polishability of cured product (Gloss) | % | 66 | 67 | 62 | 68 | 70 | 66 |
| Ease of handling | | Excellent | Good | Good | Good | Good | Good |
| Consistency stability | % | 94 | 89 | 84 | 95 | 86 | 90 |
| Polymerization shrinkage stress | MPa | 11.3 | 9.2 | 9.9 | 10.6 | 10.1 | 10.9 |

| | | | | | | | |
|---|---|---|---|---|---|---|---|
| *1)Mass fraction in polymerizable monomer composition (X) | | | | | | | |

**[Table4]**

| | | Ex. 9 | Ex. 10 | Ex. 11 | Ex. 12 | Ex. 13 | Ex. 14 | Ex. 15 | Ex. 16 |
|---|---|---|---|---|---|---|---|---|---|
| Polymerizable monomer composition (X) | Parts by mass | 25 | 25 | 25 | 25 | 25 | 25 | 25 | 25 |
| • Polymerizable monomer (A) | | | | | | | | | |
| D2.6E | Parts by mass *1) | 70 | 70 | 70 | 70 | 70 | 70 | 70 | 70 |
| Bis-GMA | Parts by mass *1) | | | | | | | | |
| 3G | Parts by mass *1) | 30 | 30 | 30 | 30 | 30 | 30 | 30 | 30 |

| • Polymerization initiator (D) | | | | | | | | | |
|---|---|---|---|---|---|---|---|---|---|
| CQ | Parts by mass *1) | 0.2 | 0.2 | 0.2 | 0.2 | 0.2 | 0.2 | 0.2 | 0.2 |
| TMDPO | Parts by mass *1) | 0.3 | 0.3 | 0.3 | 0.3 | 0.3 | 0.3 | 0.3 | 0.3 |

| • Polymerization accelerator (F) | | | | | | | | | |
|---|---|---|---|---|---|---|---|---|---|
| PDE | Parts by mass *1) | 0.2 | 0.2 | 0.2 | 0.2 | 0.2 | 0.2 | 0.2 | 0.2 |
| • Polymerization inhibitor (G) | | | | | | | | | |
| BHT | Parts by mass *1) | 0.1 | 0.1 | 0.1 | 0.1 | 0.1 | 0.1 | 0.1 | 0.1 |

| • UV absorber | | | | | | | | | |
|---|---|---|---|---|---|---|---|---|---|
| TN326 | Parts by mass *1) | 0.1 | 0.1 | 0.1 | 0.1 | 0.1 | 0.1 | 0.1 | 0.1 |

| Prepolymer (C) | | | | | | | | | |
|---|---|---|---|---|---|---|---|---|---|
| Type | | 3 | 4 | 5 | 6 | 7 | 1 | 1 | 1 |
| Content | Parts by mass | 5 | 5 | 5 | 5 | 5 | 5 | 5 | 5 |

| Inorganic filler | | | | | | | | | |
|---|---|---|---|---|---|---|---|---|---|
| SiO₂-ZrO₂-1 | Parts by mass | 70 | 70 | 70 | 70 | 70 | | | |
| SiO₂-ZrO₂-2 | Parts by mass | | | | | | 70 | | |
| SiO₂-Yb₂O₃ | Parts by mass | | | | | | | 70 | |
| SiO₂-BaO | Parts by mass | | | | | | | | 70 |
| NF180 | Parts by mass | | | | | | | | |
| UF2.0 | Parts by mass | | | | | | | | |

| Other polymer | | | | | | | | | |
|---|---|---|---|---|---|---|---|---|---|
| LA4285 | Parts by mass | | | | | | | | |

| Properties of dental composition | | | | | | | | | |
|---|---|---|---|---|---|---|---|---|---|
| Flexural strength of cured product | MPa | 118 | 124 | 127 | 120 | 118 | 132 | 130 | 115 |
| Polishability of cured product (Gloss) | % | 68 | 69 | 67 | 63 | 65 | 63 | 68 | 56 |
| Ease of handling | | Excellent | Excellent | Good | Good | Excellent | Excellent | Excellent | Good |
| Consistency stability | % | 89 | 98 | 90 | 90 | 92 | 102 | 87 | 93 |
| Polymerization shrinkage stress | MPa | 10.2 | 10.6 | 10.2 | 10.3 | 10.3 | 10.8 | 10.2 | 9.9 |

| | | | | | | | | | |
|---|---|---|---|---|---|---|---|---|---|
| *1)Mass fraction in polymerizable monomer composition (X) | | | | | | | | | |

## Claims

1. A dental composition comprising a polymerizable monomer (A), an inorganic filler (B), a prepolymer (C), and a polymerization initiator (D), the inorganic filler (B) having an average particle diameter of 1 to 20 µm, and a specific surface area of 10 to 300 m²/g.

2. The dental composition according to claim 1, wherein the prepolymer (C) comprises a structure derived from a polyfunctional monomer (a).

3. The dental composition according to claim 2, wherein the polyfunctional monomer (a) has a molecular weight of 250 to 1,000.

4. The dental composition according to claim 2 or 3, wherein the polyfunctional monomer (a) comprises at least one selected from the group consisting of a polyfunctional (meth)acrylic acid ester and a polyfunctional (meth)acrylamide.

5. The dental composition according to any one of claims 2 to 4, wherein the polyfunctional monomer (a) comprises a compound represented by the following general formula (1):
X-A-X (1),
where X is a (meth)acryloyloxy group or a (meth)acrylamide group, and A is an optionally substituted divalent aliphatic hydrocarbon group having 5 or more carbon atoms, or an optionally substituted divalent aromatic hydrocarbon group having 6 or more carbon atoms, wherein some of the constituent carbon atoms in A may be substituted with oxygen atoms and/or nitrogen atoms, and a plurality of X may be the same or different from each other.

6. The dental composition according to any one of claims 2 to 5, wherein the polyfunctional monomer (a) comprises a compound represented by the following general formula (2):
X-(R¹)ₘ-A'-(R²)ₙ-X (2),
where X is a (meth)acryloyloxy group or a (meth)acrylamide group, R¹ and R² are each independently an optionally substituted alkyleneoxy group having 1 to 6 carbon atoms, and A' is an optionally substituted divalent aliphatic hydrocarbon group having 3 or more carbon atoms, or an optionally substituted divalent aromatic hydrocarbon group having 6 or more carbon atoms, wherein some of the constituent carbon atoms in A' may be substituted with oxygen atoms and/or nitrogen atoms, m and n are each independently an integer of 1 or more, the average number of moles of alkyleneoxy groups added as represented by an average of the sum of m and n per molecule is 2 to 35, and a plurality of X, R¹, and R² each may be the same or different from each other.

7. The dental composition according to any one of claims 2 to 6, wherein the prepolymer (C) additionally comprises a structure derived from a monofunctional monomer (b).

8. The dental composition according to claim 7, wherein the monofunctional monomer (b) comprises at least one selected from the group consisting of a monofunctional (meth)acrylic acid ester and a monofunctional (meth)acrylamide.

9. The dental composition according to claim 7 or 8, wherein at least one of the polyfunctional monomer (a) and the monofunctional monomer (b) comprises an aromatic ring.

10. The dental composition according to any one of claims 7 to 9, wherein the structure derived from the polyfunctional monomer (a) and the structure derived from the monofunctional monomer (b) have a mole ratio of 10/90 to 90/10 in terms of a mole ratio of the structure derived from the polyfunctional monomer (a) to the structure derived from the monofunctional monomer (b).

11. The dental composition according to any one of claims 1 to 10, wherein the prepolymer (C) has a hydroxyl number of 250 mgKOH/g or less.

12. The dental composition according to any one of claims 1 to 11, wherein the prepolymer (C) comprises a structure derived from a chain transfer agent (c).

13. The dental composition according to any one of claims 1 to 12, wherein the inorganic filler (B) comprises a secondary particle formed by binding of inorganic primary fine particles.

14. The dental composition according to any one of claims 1 to 13, wherein the inorganic filler (B) comprises silicon dioxide and at least one metal oxide other than silicon dioxide.

15. The dental composition according to claim 14, wherein the metal oxide other than silicon dioxide is at least one selected from the group consisting of zirconium oxide, barium oxide, lanthanum oxide, and ytterbium oxide.

16. The dental composition according to any one of claims 1 to 15, wherein the inorganic filler (B) is surface treated with a silane coupling agent.

17. The dental composition according to any one of claims 1 to 16, wherein the dental composition comprises 10 to 97 mass% of the inorganic filler (B).

18. The dental composition according to any one of claims 1 to 17, wherein the dental composition comprises 0.5 to 20 mass% of the prepolymer (C).

19. The dental composition according to any one of claims 1 to 18, wherein the dental composition is a dental composite resin.
